# EUROPEAN PATENT APPLICATION

(11) **EP 4 653 440 A1**
(43) Date of publication of application: **26.11.2025**
(21) Application number: 24744322.9
(22) Date of filing: 17.01.2024
(51) Int. Cl.: C07D 487/04, A61K 31/519, A61P 27/06, A61P 35/00, A61P 11/06, A61P 5/48, A61P 13/12, A61P 19/10, A61P 25/28

(54) **HETEROCYCLIC COMPOUND, AND PHARMACEUTICAL COMPOSITION AND USE THEREOF**

(30) Priority: 18.01.2023 CN 202310095802
(71) Applicant: Prime Gene Therapeutics Co., Ltd., Beijing 100070 (CN)
(72) Inventor: ZHU, Li, Beijing 100070 (CN); HU, Wei, Beijing 100070 (CN); ZHANG, Hui, Beijing 100070 (CN); DU, Daniel Yunlong, Beijing 100070 (CN)
(74) Representative: Winter, Brandl - Partnerschaft mbB
(86) International application number: PCT/CN2024/072857
(87) International publication number: WO 2024/153148

(57) **Abstract**

The present application relates to a heterocyclic compound, a pharmaceutical composition and use thereof. The heterocyclic compound has a structural formula as shown in formula (I). The heterocyclic compound provided in the present invention has significant Rho kinase inhibitory activity, and the enzymatic activity and cell activity thereof are better than those of existing ROCK inhibitors such as Ripasudil, Netarsudil and belumosudil, thereby the heterocyclic compound has great application potential.

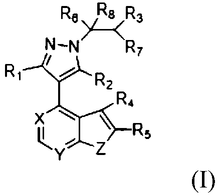

## Description

### Technical Field

The present application provides a heterocyclic compound, a stereoisomer and a pharmaceutical composition comprising the compound, and use of the compound and the pharmaceutical composition. The heterocyclic compound is an inhibitor targeting ROCK that can be used to regulate Rho kinase mediated diseases.

### Background

Rho/ROCK (Rho associated kinase) signaling pathway induces cytoskeleton reorganization, cell migration, adhesion and stress fiber formation, which are related to various physiological functions. ROCK family includes ROCK1 and ROCK2. ROCK1 is highly expressed in lung, liver, spleen, kidney and testis, while ROCK2 is highly expressed in brain and heart. ROCK1 indirectly acts on an epithelial cadherin compound by binding to the scaffold protein P120 catenin of E-cadherin; ROCK1 can be concentrated in the microtubule tissue center and the pseudopodia edge of motor cells, which is related to cell migration. ROCK2 is mainly concentrated in cytoplasm and is located on plasma membrane through C-terminal region, which is related to vimentin and actin tension fibers.

ROCK mediates many pathological and physiological signals, which are related to various physiological functions such as endothelial permeability, tissue contraction, and growth. ROCK inhibitors have potential applications in diseases comprsing asthma, cancer, glaucoma, insulin resistance, kidney failure, neuronal degeneration, and osteoporosis. Two ROCK1/ROCK2 inhibitors Ripasudil and Netarsudil eye drops have been approved for the treatment of glaucoma, and one ROCK2 inhibitor belumosudil has been approved for the treatment of chronic graft-versus-host disease (cGVHD). However, the existing ROCK inhibitors on the market have weak activity, and the function and clinical application value of ROCK have not been fully developed. Therefore, it is urgent to develop a pharmaceutical targeting ROCK to address the unmet clinical needs. The present application developes a highly active heterocyclic compound targeting ROCK with a novel framework and a pharmaceutical composition thereof for regulating Rho kinase mediated diseases.

### Summary of the Invention

On the one hand, the present disclosure provides a compound shown in formula I, isotope isomers or pharmaceutically acceptable salts, solvates, active metabolites, polymorphs, isotopic markers, isomers or prodrugs thereof:

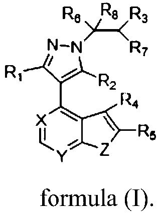

Another aspect of the present disclosure is to provide a method for manufacturing the compound as shown in formula I, isotope isomers or pharmaceutically acceptable salts, active metabolites, polymorphs, isotopic markers, isomers or prodrugs thereof, and use thereof for treating Rho kinase mediated diseases.

### Brief description of the drawings

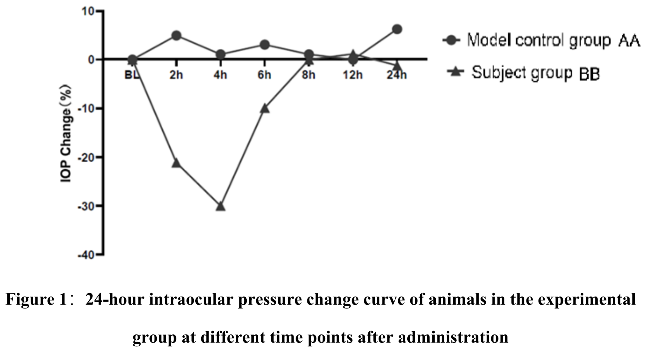

Figure 1 shows the 24-hour intraocular pressure change curve of animals in the experimental group at different time points after administration.

### Detailed Description

The present application will be further explained below in detail through embodiments. Through these descriptions, the features and advantages of the present application will become more clearly defined.

The term "exemplary" used here means "serving as an example, embodiment, or illustration." Any embodiment described here as "exemplary" should not be interpreted as being superior or better than other embodiments.

Furthermore, the technical features in different embodiments of the present application described below can be combined with each other if they do not conflict.

### Definitions

Unless defined otherwise, all technical and scientific terms used herein have the same meaning as that commonly understood by one of skill in the art to which the claimed subject matter belongs. All patents, patent applications, published materials referred to throughout the entire disclosure herein, unless noted otherwise, are incorporated by reference in their entirety. When a trade name appears herein, it is intended to refer to its corresponding product or its active ingredient.

It is to be understood that the foregoing general description and the following detailed description are exemplary and explanatory only, and are not restrictive of any subject matter claimed. In the present application, it must be noted that unless otherwise explicitly stated in the text, the singular used in the present description and claims includes the plural of the referred object. It should also be noted that "or" used herein means "and/or" unless stated otherwise. Furthermore, the term "comprise(s)" used herein as well as other forms, such as "comprising", "contain(s)", and "include(s)" is not limiting.

Definitions of standard chemical terms may be found in the literature, including Carey and Sundberg's "Advanced Organic Chemistry 4th Ed, Vol A (2000) and B (2001), Plenum Press, New York. Unless otherwise specified, conventional methods within the technical field are applied, such as mass spectrometry, NMR, HPLC, protein chemistry, biochemistry, recombinant DNA technology, and pharmacological methods. Unless specific definitions are provided, those skilled in the art know the related terms and laboratory operations and techniques in analytical chemistry, synthetic organic chemistry, and medical and pharmaceutical chemistry used herein. Standard techniques may be used for chemical synthesis, chemical analysis, drug preparation, formulation, drug delivery and patient treatment. Standard techniques may be used for recombinant DNA, oligonucleotide synthesis, and tissue culture and transformation (such as electroporation, lipid infection). For example, a kit with instructions provided by the manufacturer may be used, or the reaction and purification techniques may be carried out according to methods known in the art, or according to the method described in the present disclosure. Generally speaking, the aforementioned techniques and steps may be implemented by conventional methods well-known in the art and described in various general documents or more specific documents. These documents are described and cited in the present disclosure.

When a substituent is described by a conventional chemical formula written from left to right, the substituent also includes chemically equivalent substituents obtained when the structural formula is written from right to left. For example, CH₂O is equivalent to OCH₂.

The term "substituted or unsubstituted" comprsies two situations, i.e. "substituted" and "unsubstituted", wherein "substituted" means that any one or more hydrogen atoms on a specific atom are replaced by a substituent, as long as the valence of the specific atom is normal and the compound after substitution is stable; "unsubstituted" means that the hydrogen atom on a specific atom has not been replaced by a substituent. For example, "substituted or unsubstituted ethyl" (such as in the case of halogen substituents) comprsies unsubstituted (-CH₂CH₃), monosubstituted (such as -CH₂CH₂F), multi-substituted (such as -CHFCH₂F, -CH₂CHF₂, etc.), or completely substituted (-CF₂CF₃). Those skilled in the art can understand that for any group containing one or more substituents, no substitution or substitution pattern that is spatially impossible and/or cannot be synthesized will be introduced. When the substituent is oxo (i.e. =O), it means that two hydrogen atoms on the same atom are replaced.

When any variable (such as R) occurs more than once in the composition or structure of a compound, its definition in each case is independent. Thus, for example, if a group is substituted with 0-2 R, the group may optionally be substituted with up to two R, and R has independent options in each case. In addition, combinations of substituents and/or variants thereof are only permitted if such combinations result in stable compounds. The term "optional" or "optionally" means that the subsequently described event or circumstance may or may not occur, and that the description includes instances wherein said event or circumstance occurs and instances in which it does not.

As used herein, C_{m~n} refers to the part having m~n carbon atoms. For example, the "C_{1~8}" group means that the part has 1-8 carbon atoms, that is, the group contains 1 carbon atom, 2 carbon atoms, 3 carbon atoms... 8 carbon atoms. Therefore, for example, "C_{1~8} alkyl" refers to an alkyl containing 1-8 carbon atoms, that is, the alkyl is selected from the group consisting of methyl, ethyl, propyl, isopropyl, n-butyl, isobutyl, sec-butyl, tert-butyl... octyl, etc. Numerical ranges herein, for example "1-8" refers to each integer within the given range. For example, "1-8 carbon atoms" means that the group may have 1 carbon atom, 2 carbon atoms, 3 carbon atoms, 4 carbon atoms, 5 carbon atoms, 6 carbon atoms, 7 carbon atoms, or 8 carbon atoms.

The term "alkyl" refers to an optionally substituted straight-chain or optionally substituted branched-chain saturated aliphatic hydrocarbon radical, which is connected to the rest of the molecule through a single bond. The "alkyl" herein may have from 1 to about 8 carbon atoms, for example, from 1 to 6 carbon atoms, or from 1 to 4 carbon atoms, or from 1 to 3 carbon atoms. Examples of the alkyl herein include but not limited to methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, n-butyl, isobutyl, sec-butyl, tert-butyl, n-pentyl, isopentyl, neopentyl, tert-amyl, hexyl, etc, and longer alkyl, such as heptyl, octyl and the like. The group defined herein, when the number range of "alkyl" appears, for example, "C₁₋₈ alkyl" refers to the alkyl which consists of 1 carbon atom, 2 carbon atoms, 3 carbon atoms, 4 carbon atoms, 5 carbon atoms, 6 carbon atoms, 7 carbon atoms, or 8 carbon atoms. As another example, "C₁₋₄ alkyl" refers to the alkyl which consists of 1 carbon atom, 2 carbon atoms, 3 carbon atoms, or 4 carbon atoms. The alkyl herein also includes cases where the numerical range is not specified.

The term "alkenyl" refers to an optionally substituted straight-chain or optionally substituted branched-chain monovalent hydrocarbon group which contains at least one carbon-carbon double bond. The alkylene has, but not limited to, from 2 to about 8 carbon atoms, for example, from 2 to about 6 carbon atoms, or from 2 to about 4 carbon atoms. The group may be in either cis or trans conformation about the double bond(s), and should be understood to include both isomers. Examples of alkylene include, but are not limited to ethenyl (CH=CH₂), 1-propenyl (CH₂CH=CH₂), isopropenyl (C(CH₃)=CH₂), butenyl, 1,3-butadienyl and the like. When the number range of the alkenyl defined herein appears, for example, "C₂₋₈ alkenyl" refers to the alkenyl which may consist of 2 carbon atoms, 3 carbon atoms, 4 carbon atoms, 5 carbon atoms, 6 carbon atoms, 7 carbon atoms, or 8 carbon atoms. The alkenyl herein also includes cases where the numerical range is not specified.

The term "alkynyl" refers to an optionally substituted straight- or branched-chain monovalent hydrocarbon group which has at least one carbon-carbon triple-bond. The alkynyl has from 2 to about 8 carbon atoms, for example, from 2 to about 6 carbon atoms, or from 2 to about 4 carbon atoms. Examples of the alkynyl herein include, but are not limited to ethynyl, 2-propynyl, 2-butynyl, 1,3-butadiynyl, and the like. When the number range of the alkynyl defined herein appears, for example, "C₂₋₈ alkynyl" refers to the alkynyl which may consist of 2 carbon atoms, 3 carbon atoms, 4 carbon atoms, 5 carbon atoms, 6 carbon atoms, 7 carbon atoms, or 8 carbon atoms. The alkynyl herein also includes cases where the numerical range is not specified.

The term "cycloalkyl" refers to non-aromatic carbon containing rings, which comprise saturated carbon rings (such as cycloalkyl) or unsaturated carbon rings (such as cycloalkenyl). Carbocyclic ring comprsies mono-carbocyclic ring (having one ring), such as monocyclic cycloalkyl; bi-carbocyclic ring (having two rings), such as bicyclic cycloalkyl; poly-carbocyclic ring (having two or more rings). The rings can be bridged or sprio rings. Carbocyclic ring (such as cycloalkyl or cycloalkenyl) can have from 3 to 8 carbon atoms, such as from 3 to about 6 ring-forming carbon atoms, or form 3 to about 5 ring-forming carbon atoms.

The term "aryl" refers to an optionally substituted aromatic hydrocarbon group which has 6 to about 20, for example, 6 to 12 or 6 to 10 ring-forming carbon atoms, which may be monocyclic aryl, bicyclic aryl or polycyclic aryl. The bicyclic aryl or polycyclic aryl may be fused through a monocyclic aryl with other independent rings, such as alicyclic, heterocyclic, aromatic, heteroaromatic rings. Non-limiting examples of the monocyclic aryl group comprise monocyclic aryl having from 6 to about 12, from 6 to about 10, or from 6 to about 8 ring-forming carbon atoms, for example, phenyl; bicyclic aryl, for example, naphthyl; and polycyclic aryl, for example, phenanthrenyl, anthracenyl, and azulenyl.

The term "heteroaryl" refers to an optionally substituted heteroaryl containing from about 5 to about 20, for example, from 5 to 12, or from 5 to 10 skeletal ring-forming atoms, wherein at least one (for example, 1 to 4, 1 to 3, or 1 to 2) ring-forming atom is a heteroatom which is independently selected from the group consisting of oxygen, nitrogen, sulfur, phosphorous, silicon, selenium and tin, but not limited to these atoms. Heteroaryl comprises monocyclic heteroaryl (containing one ring), bicyclic heteroaryl (containing two rings) or polycyclic heteroaryl (containg more than two rings). In embodiments in which two or more heteroatoms are present in the ring, the two or more heteroatoms can be identical to each another, or some or all of the two or more heteroatoms can each be different from the others. The bicyclic heteroaryl or polycyclic heteroaryl may be fused through a monocyclic heteroaryl with other independent rings such as alicyclic, heterocyclic, aromatic, heteroaromatic rings (which may be collectively known as fused ring heteroaryl). Non-limiting examples of heteroaryl comprise but are not limited to pyrrolyl, furyl, thienyl, imidazolyl, oxazolyl, pyrazolyl, pyridinyl, pyrimidyl, pyrazinyl, quinolyl, isoquinolyl, tetrazolyl, triazolyl, triazinyl, benzofuranyl, benzothienyl, indolyl, isoindolyl, and the like.

The term "heterocyclyl" refers to a non-aromatic heterocycle, which comprises a saturated heterocycle or an unsaturated heterocycle (containing an unsaturated bond), does not have a completed conjugated π-electron system and can be divided into non-aromatic monocyclic, fused polycyclic, bridged or spirocyclic systems, wherein one or more (e.g., 1 to 4, 1 to 3, or 1 to 2) ring-forming atoms are heteroatoms such as oxygen, nitrogen or sulfur atoms. The heterocycle may comprise mono-heterocycle (containing one ring) or bis-heterocycle (containing two bridged rings) or poly-heterocycle (containing more than two bridged rings); and also comprise sprio rings. Heterocyclyl may have from 3 to about 20, for example, from 3 to about 10, from 3 to about 8, from 4 to 8, from 4 to 7, from 5 to about 8, or from 5 to about 6 ring atoms. Non-limiting examples of "heterocyclyl" comprise oxiranyl, thiiranyl, aziranyl, azetidinyl, oxetanyl, thietanyl, tetrahydrofuranyl, pyrrolidinyl, oxazolidinyl, tetrahydropyrazolyl, pyrrolinyl, dihydrofuranyl, dihydrothienyl, piperidinyl, tetrahydropyranyl, tetrahydrothiapyranyl, morpholinyl, piperazinyl, dihydropyridyl, tetrahydropyridyl, dihydropyranyl, tetrahydropyranyl, dihydrothiapyranyl, azacycloheptyl, oxacycloheptyl, thiacycloheptyl, oxa-azabicyclo[2.2.1]heptyl, azaspiro[3.3]heptyl, and the like.

The term "halo" or "halogen" refers to an optionally substituted group (such as alkyl, alkenyl, alkynyl, etc.), in which at least one hydrogen atom is replaced with a halogen atom (such as fluorine, chlorine, bromine, iodine, or a combination thereof). In some embodiments, two or more hydrogen atoms are replaced with halogen atoms that are identical to each other (e.g., difluoromethyl, trifluoromethyl); in other embodiments, two or more hydrogens are replaced with halogen atoms that are not completely identical to each other (e.g., 1-chloro-1-fluoro-1-iodo-ethyl).

The term "alkoxy" refers to an alkyl ether group (O-alkyl). Non-limiting examples of alkoxy radicals comprise methoxy, ethoxy, n-propoxy, isopropoxy, n-butoxy, isobutoxy, sec-butoxy, tert-butoxy and the like.

The term "alkylacyl" refers to a group formed by connecting an alkyl with -CO-, and non-limiting examples thereof include formyl, acetyl, propionyl, butyryl, and the like. For example, the term "C₁₋₆ alkylacyl" refers to a group formed by connecting C₁₋₆ alkyl with -CO-. As another example, the term "C₁₋₄ alkylacyl" refers to a group formed by connecting C₁₋₄ alkyl with -CO-.

The term "alkylsulfonyl" refers to a group formed by connecting an alkyl with -SO₂-, and non-limiting examples thereof comprise methylsulfonyl, ethylsulfonyl, propylsulfonyl, butylsulfonyl, and the like. For example, the term "C₁₋₆ alkylsulfonyl" refers to a group formed by connecting C₁₋₆ alkyl with -SO₂-. As another example, the term " C₁₋₄ alkylsulfonyl" refers to a group formed by connecting C₁₋₄ alkyl with -SO₂-.

The term "heteroarylacyl" refers to a group formed by connecting a heteroaryl with-CO-. For example, the term "C₅₋₂₀ heteroarylacyl" refers to a group formed by connecting C₅₋₂₀ heteroaryl with -CO-. The "heteroaryl" and "C₅₋₂₀ heteroaryl" are defined as above.

The terms "monocyclic", "monocyclyl ", or "monocyclic ring system" refer to the structure of one ring (e.g., cycloalkyl, cycloalkynyl, aryl, and/or heterocyclic groups). The terms "polycyclyl", "polycyclic group" or "polycyclic system" refer to two or more rings (e.g., cycloalkyl, cycloalkenyl, cycloalkynyl, aryls and/or heterocyclyl), in which two adjacent rings share one, two, or more ring atoms, for example, the polycyclic ring can be "fused ring", "spiro ring", or bridged ring structure.

The terms "bicyclyl carbon ring" refer to an aromatic or non-aromatic ring containing two rings, where each atom in the ring is carbon, and the two rings share one, two, or more ring atoms. For example, the rings are "fused rings" or " spiro ring". Rings that are joined through non-adjacent atoms are termed "bridged" rings, for example C₅-C₁₂ bridged carbon ring, comprsing but not limited to bicyclic [2.2.2] octane, bicyclic [1.1.1] pentane, bicyclic [3.2.1] octane, and bicyclic [2.1.1] hexane. Each ring in the polycycle can be substituted with such substituents as described above, for example, halogen, alkyl, aralkyl, alkenyl, alkynyl, cycloalkyl, hydroxyl, amino, nitro, mercapto, imino, amido, phosphate, phosphonate, phosphonite, carbonyl, carboxyl, silyl, ether, alkylthio, sulfonyl, ketone, aldehyde, ester, heterocyclyl, aromatic or heteroaromatic moiety, -CF₃, -CN, or the like. The term " C₅-C₁₂ bicyclic carbon ring" refers to a bicyclic carbon ring with 5-12 carbon atoms on the ring, excluding the carbon atoms on its substituents. It can include a C₅-C₁₂ spirocyclic carbon ring, a C₅-C₁₂ fused carbon ring, or a C₅-C₁₂ bridged carbon ring.

Other terms for group herein comprise: "hydroxyl" refers to -OH group, "thiol" refers to - SH group, "cyano" refers to -CN group, and "carboxyl" refers to -COOH group.

The term "member" refers to the number of skeletal atoms constituting the ring. For example, pyridine is a six-membered ring and pyrrole is a five-membered ring.

The term "pharmaceutically acceptable" refers to those compounds, materials, compositions and/or dosage forms that are within the scope of reliable medical judgment and are suitable for use in contact with human and animal tissues without excessive toxicity, irritation, allergic reactions or other problems or complications of the disease, and are commensurate with a reasonable benefit/risk ratio.

The term "pharmaceutical composition" refers to a biologically active compound optionally mixed with at least one pharmaceutically acceptable chemical component or agent. The pharmaceutically acceptable chemical component or agent is the "carrier", which helps for introducing the compound into cells or tissues. It comprises, but is not limited to, stabilizers, diluents, suspending agents, thickeners, and/or excipients.

The term "pharmaceutically acceptable salt" refers to a salt that retains the biological efficacy of the free acid and free base of the specified compound and has no adverse effects in biology or other aspects. Unless otherwise specified, the salts in the present disclosure may comprise metal salts, ammonium salts, salts formed with organic bases, salts formed with inorganic acids, salts formed with organic acids, salts formed with basic or acidic amino acids, etc. Non-limiting examples of metal salts comprise, but are not limited to, alkali metal salts, such as sodium salt, potassium salt, etc.; alkaline earth metal salts, such as calcium salt, magnesium salt, barium salt, etc.; aluminum salt, and the like. Non-limiting examples of salts formed with organic bases comprise, but are not limited to, the salts formed with trimethylamine, triethylamine, pyridine, picoline, 2,6-lutidine, ethanolamine, diethanolamine, triethanolamine, cyclohexylamine, dicyclohexylamine and the like. Non-limiting examples of salts formed with inorganic acids comprise, but are not limited to, salts formed with hydrochloric acid, hydrobromic acid, nitric acid, sulfuric acid, phosphoric acid, and the like. Non-limiting examples of salts formed with organic acids comprise, but are not limited to, salts formed with formic acid, acetic acid, trifluoroacetic acid, fumaric acid, oxalic acid, malic acid, maleic acid, tartaric acid, citric acid, succinic acid, methanesulfonic acid, benzene sulfonic acid, p-toluenesulfonic acid, etc. Non-limiting examples of salts formed with basic amino acids comprise, but are not limited to, salts formed with arginine, lysine, ornithine, and the like. Non-limiting examples of salts formed with acidic amino acids comprise, but are not limited to, salts formed with aspartic acid, glutamic acid, and the like.

Pharmaceutically acceptable salts may be synthesized from parent compounds containing acid radicals or bases by conventional chemical methods. Generally, such salts are prepared by reacting these compounds in free acid or base form with a stoichiometric amount of appropriate base or acid in water or organic solvent or a mixture of both. Generally, non-aqueous media such as ether, ethyl acetate, ethanol, isopropanol or acetonitrile are preferred.

The term "solvate" refers to a physical aggregate formed by a compound of the present imvention and one or more solvent molecules. This physical aggregate comprises varying degrees of ions and covalent bonds, such as hydrogen bonds. It has been shown that this solvate may be separated, for example, when one or more solvent molecules are mixed in the crystal lattice. "solvate" comprises both solvent phase and separable solvate. There are many examples of corresponding solvates, including ethanol solvates, methanol solvates and the like. "Hydrate" is a solvate that uses water (H₂O) molecules as a solvent. One or more compounds in the present disclosure may be prepared as solvates at will. The preparation of solvates is well known. For example, M. Caira et al, J. Pharmaceutical Sci., 93(3), 601-611 (2004 ) describe the preparation of a solvate of the antifungal drug fluconazole, that is, preparation with ethyl acetate and water. E.C. van Tonder et al, AAPS PharmSciTech., 5(1), article 12 (2004); and AL Bingham et al, Chem. Commun., 603-604 (2001) also describes the similar methods for preparing solvates and hydrates. A typical, non-limiting preparation process is to dissolve the compound of the present disclosure in an ideal solvent (organic solvent or water or their mixed solvent) at a temperature higher than normal temperature, to cool down, and to leave to crystallize. Then, the crystals are separated by using standard methods. I. R. spectroscopy analysis technique may confirm the existence of the solvent (water) that forms the solvate (hydrate) in the crystal.

The term "active metabolite" refers to an active derivative of the compound formed when the compound is metabolized.

The term "polymorphs" refers to compounds of the present disclosure that exist in different crystal lattice forms.

The term "isotopic marker" refers to an isotopically marked compound of the present disclosure. For example, the isotopes in the compound of the present disclosure may comprise various isotopes of elements such as H, C, N, O, P, F, S, such as ²H, ³H, ¹³C, ¹⁴C, ¹⁵N, ¹⁸O, ¹⁷O, ³¹P, ³²P, ³⁵S, ¹⁸F and ³⁶S.

The term "pharmaceutically acceptable prodrug" or "prodrug" refers to any pharmaceutically acceptable salt, ester, ester salt or other derivative of the compound of the present disclosure, which may directly or indirectly provide the compound of the present disclosure or its pharmaceutically active metabolite or residue after administration to a recipient. Particularly preferred derivatives or prodrugs are those compounds that may improve the bioavailability of the compounds of the present disclosure when administered to patients (for example, may make oral compounds more easily absorbed into the blood), or promote the parent compound delivering to biological organs or the site of action, such as the brain or lymphatic system. The functional groups in the compound may be modified by conventional operations or in vivo in a modification manner that may be decomposed into the parent compound to prepare a prodrug. Various prodrug forms are well known in the art. See, Pro-drugs as Novel Delivery Systems (1987) Vol. 14 of the ACS Symposium Series, Bioreversible Carriers in Drug Design, (1987) Edward B. Roche, ed., American Pharmaceutical Association by T. Higuchi and V. Stella and Pergamon Press provide discussions on prodrugs. Design of Prodrugs, Bundgaard, A. Ed., Elseview, 1985 and Method in Enzymology, Widder, K. et al., Ed.; Academic, 1985, vol. 42, p. 309-396 ; Bundgaard, H. "Design and Application of Prodrugs" in A Textbook of Drug Design and Development, Krosgaard-Larsen and H. Bundgaard, Ed., 1991, Chapter 5, pp. 113-191 ; and Bundgaard, H., Advanced Drug Delivery Review, 1992, 8, 1-38 , the above documents are incorporated herein by reference.

The term "stereoisomers" refers to isomers produced by different arrangements of atoms in the molecule in space. The compounds of the present disclosure contain structures such as asymmetric or chiral centers and double bonds. Therefore, the compounds of the present disclosure may comprise optical isomers, geometric isomers, tautomers, atropisomers and other isomers. These isomers and their single isomers, racemates, etc. are all included in the scope of the present disclosure. For example, for optical isomers, optically active (R)- and (S)-isomers and D and L isomers may be prepared by chiral resolution, chiral synthesis or chiral reagents or other conventional techniques. For example, it may be converted into diastereomers by reacting with appropriate optically active substances (such as chiral alcohols or Mosher's Mohsyl chloride), separated and converted (such as hydrolyzed) into the corresponding single isomer. As another example, it may also be separated by a chromatographic column.

The "pharmaceutical compositions" herein may be prepared in a manner well known in the pharmaceutical field, and they may be administered or applied by various routes, depending on whether local or systemic treatment is required and the area to be treated. It may be topically administered (for example, transdermal, skin, eye and mucous membranes including intranasal, vaginal and rectal delivery), pulmonarily administered (for example, by inhalation or insufflation of powder or aerosol, including through sprayers; intratracheal, intranasal), orally or parenterally administered. Parenteral administration includes intravenous, intraarterial, subcutaneous, intraperitoneal or intramuscular injection or infusion; or intracranial, such as intrathecal or intracerebroventricular administration. It may be administered parenterally in a single bolus dose, or it may be administered by, for example, a continuous infusion pump. The pharmaceutical composition herein comprises but is not limited to the following forms: tablets, pills, powders, lozenges, sachets, cachets, elixirs, suspensions, emulsions, solutions, syrups, aerosols (solid or dissolved in a liquid vehicle); for example, ointments, soft and hard gelatin capsules, suppositories, sterile injection solutions and sterile packaged powders containing up to 10% by weight of the active compound.

The pharmaceutical composition herein may be formulated in a unit dosage form, and each dosage may contain about 0.1 to 1000 mg, usually about 5 to 1000 mg of active ingredient, and more usually about 100 to 500 mg of active ingredient. The term "unit dosage form" refers to a physically separated single dosage unit suitable for use in human patients and other mammals, each unit containing a predetermined amount of active substance mixed with a suitable pharmaceutical carrier calculated to produce the desired therapeutic effect.

The term "individual" refers to an individual suffering from a disease, disorder, or condition, and comprises mammals and non-mammals. Examples of mammals comprise, but are not limited to, any member of the class Mammals: humans, non-human primates (such as chimpanzees and other apes and monkeys); domestic animals, such as cows, horses, sheep, goats, and pigs; domesticated animals, such as rabbits, dogs, and cats; laboratory animals, comprising rodents, such as rats, mice, and guinea pigs.

The term "treatment" and other similar synonyms comprise alleviation, reduction or amelioration of symptoms of a disease or condition, prevention of other symptoms, amelioration or prevention of the underlying metabolic cause of the symptoms, inhibition of the disease or condition, such as preventing the development of the disease or condition, alleviating the disease or condition, making a disease or condition better, alleviating the symptoms caused by the disease or condition, or stoping the symptoms of the disease or condition. In addition, the term may also comprise the purpose of prevention. The term also comprises obtaining therapeutic effects and/or preventive effects. The therapeutic effect refers to curing or improving the underlying disease being treated. In addition, the cure or improvement of one or more physiological symptoms associated with the underlying disease is also a therapeutic effect. For example, although the patient may still be affected by the underlying disease, an improvement in the patient's condition is observed. In terms of preventive effects, the composition or compound may be administered to patients who are at risk of suffering from a specific disease, or even if a disease diagnosis has not been made, the composition or compound may be administered to patients who have one or more physiological symptoms of the disease.

The term "amount to obtain the necessary therapeutic effect" or "therapeutically effective amount" refers to the amount of at least one agent or compound sufficient to relieve one or more symptoms of the disease or condition being treated after administration. The result may be a reduction and/or alleviation of signs, symptoms or causes, or any other desired changes in the biological system. Techniques such as dose escalation tests may be used to determine the effective amount suitable for any individual case. The actual amount of compound, pharmaceutical composition or agent to be administered is usually determined by the physician according to the relevant circumstances, comprising the condition being treated, the route of administration selected, the actual compound administered; the age, weight and response of the individual patient; and the severity of the patient's symptoms, etc.

The ratio or concentration of the compound of the present disclosure in the pharmaceutical composition may not be fixed, depending on various factors, comprising dosage, chemical properties (for example, hydrophobicity), route of administration, and the like. For example, the compound of the present disclosure may be provided by a physiologically buffered aqueous solution containing about 0.1-10% w/v of the compound for parenteral administration. Some typical dosage ranges are from about 1 µg/kg to about 1 g/kg body weight/day. In certain embodiments, the dosage range is from about 0.01 mg/kg to about 100 mg/kg body weight/day. The dosage is likely to depend on such variables, such as the type and degree of development of the disease or condition, the general health status of the specific patient, the relative biological efficacy of the selected compound, the excipient formulation and its route of administration.

The term "administration" refers to a method capable of delivering a compound or composition to a desired site for biological action. These methods comprise, but are not limited to, oral routes, transduodenal routes, parenteral injections (comprising intravenous, subcutaneous, intraperitoneal, intramuscular, intraarterial injection or infusion), topical, and rectal administration. Those skilled in the art are familiar with administration techniques that may be used for the compounds and methods described herein, for example, those discussed in Goodman and Gilman, The Pharmacological Basis of Therapeutics, currented.; Pergamon; and Remington's, Pharmaceutical Sciences (current edition), Mack Publishing Co., Easton, Pa.

The term "IC₅₀" refers to 50% inhibition of the maximum effect obtained in an analysis measuring such an effect.

### Compounds

The present invention provides a heterocyclic compound shown in formula (I), or pharmaceutically acceptable salts, solvates, active metabolites, polymorphs, isotopic markers, isomers or prodrugs thereof: 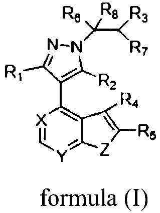 wherein X, Y and Z are each independently selected from the group consisting of C and N atoms, and X, Y and Z are not all C atoms at the same time;
R₁ is -NHR₁₁, -OR₁₁, -SR₁₁, or -C(=O)NHR₁₁; wherein R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C_{5~20} heteroaryl, substituted or unsubstituted C_{1~6} alkylacyl, substituted or unsubstituted C_{1~6} alkylsulfonyl, substituted or unsubstituted C₅₋₂₀ heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, -C(=O)R₃₃, hydroxyl, carboxyl, mercapto, substituted C_{3~8} cycloalkyl, substituted C_{3~8} heterocyclyl, substituted C_{6~20} aryl, and substituted C_{5~20} heteroaryl;
R₂ is selected from the group consisting of hydrogen, halogen, substituted or unsubstituted C_{1~6} alkyl, substituted or unsubstituted C₂₋₆ alkenyl, substituted or unsubstituted C₂₋₆ alkynyl, substituted or unsubstituted C_{1~6} haloalkyl, substituted or unsubstituted C_{1~6} alkoxy, cyano, amino, hydroxyl, carboxyl, and mercapto; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃ is selected from the group consisting of cyano, -CONH₂, and carboxyl;
R₄ and R₅ are each independently selected from the group consisting of hydrogen, halogen, -NR₃₁R₃₂, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
wherein (i) R₆ and R₈ are each independently selected from the group consisting of hydrogen, substituted or unsubstituted cyclopropyl, substituted or unsubstituted cyclobutyl, substituted or unsubstituted cyclohexyl, and substituted or unsubstituted bicyclic carbon ring; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, oxo, carboxyl, and mercapto; and R₆ and R₈ are not all hydrogen;
or, (ii) R₆ and R₈, together with the C atom to which R₆ and R₈ are connected, form a 4-to 8-membered monocyclic or polycyclic ring system; the 4- to 8-membered monocyclic or polycyclic ring system is optionally substituted with one or more R₄₁;
R₇ is selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{1~6} haloalkyl, substituted or unsubstituted C_{1~6} alkoxy, amino, hydroxyl, carboxyl, and mercapto;
or, R₆ is hydrogen, R₈ and the C atom to which R₈ is connected, together with R₇ and the C atom to which R₇ is connected, form a 5- to 8-membered carbon ring; the 5- to 8-membered carbon ring is optionally substituted with one or more R₄₁;
R₃₁ and R₃₂ are each independently selected from the group consisting of hydrogen, sulfonamido, substituted or unsubstituted C_{1~6} alkyl, substituted or unsubstituted C₂₋₆ alkenyl, substituted or unsubstituted C₂₋₆ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C₅₋₂₀ heteroaryl, cyano, hydroxyl, -C(=O)OR₃₄, and mercapto;
R₃₃ is selected from the group consisting of hydrogen, substituted or unsubstituted C_{1~8} alkyl, substituted or unsubstituted C_{1~8} alkoxy, substituted or unsubstituted C_{3~8} cycloalkyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃₄ is selected from the group consisting of hydrogen, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto;
R₄₁ is selected from the group consisting of hydrogen, halogen, oxo, cyano, hydroxyl, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto.

It should be noted that in the present invention, when X, Y, and Z are C or N atoms, the C or N atom can also be connected to hydrogen atoms that satisfy the required number of valence bonds for the C or N atom. For example, when Z is N atom, the N atom is also connected to hydrogen atoms that satisfy the N atom valence bond number; when X and Y are N atoms, the N atoms do not need to be connected to any hydrogen atom. In one embodiment, X and Y are N atoms, and Z is C atom or N atom. In another embodiment, X, Y, and Z are all N atoms.

In one embodiment, R₁ is selected from the group consisting of -NHR₁₁, wherein R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C₅₋₂₀ heteroaryl, substituted or unsubstituted C_{1~6} alkylacyl, substituted or unsubstituted C_{1~6} alkylsulfonyl, substituted or unsubstituted C_{5~20} heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, -C(=O)R₃₃, hydroxyl, mercapto, substituted C_{3~8} cycloalkyl, substituted C_{3~8} heterocyclyl, substituted C_{6~20} aryl, and substituted C_{5~20} heteroaryl. The inventor found that when R₁ is -NHR₁₁, there is one active hydrogen on R₁, which can enhance the ROCK biological activity of the compound.

In one embodiment, R₁₁ is selected from the group consisting of hydrogen, substituted or unsubstituted C_{1~6} alkyl, the substituent is selected from the group consisting of -NR₃₁R₃₂, - C(=O)R₃₃, and substituted or unsubstituted C_{3~8} heterocyclyl. In one embodiment, R₁₁ is selected from the group consisting of -NHR₁₁, R₁₁ can be selected from the group consisting of hydrogen, optionally substituted methyl, optionally substituted ethyl, optionally substituted propyl, optionally substituted isopropyl, optionally substituted butyl, optionally substituted tert-butyl, optionally substituted pentyl, optionally substituted hexyl, etc., the substituent can be selected from the group consisting of -NR₃₁R₃₂, -C(=O)R₃₃, and substituted or unsubstituted C_{3~8} heterocyclyl (the substituent can be C_{1~6} alkyl, such as methyl, ethyl, propyl, isopropyl, etc), and the like. Preferably, R₁₁ is selected from the group consisting of substituted or unsubstituted C_{1~6} alkyl, the substituent is selected from the group consisting of -NR₃₁R₃₂, wherein R₃₁ and R₃₂ are each independently hydrogen, substituted or unsubstituted C_{1~6} alkyl, or substituted or unsubstituted C_{3~8} heterocyclyl (the substituent can be C_{1~6} alkyl, such as methyl, ethyl, propyl, isopropyl, etc). Preferably, R₁₁ is selected from the group consisting of substituted or unsubstituted C_{1~6} alkyl, the substituent is selected from the group consisting of -C(=O)R₃₃, wherein R₃₃ is independently substituted or unsubstituted C_{1~6} alkyl, or substituted or unsubstituted C_{3~8} heterocyclyl (the substituent can be C_{1~6} alkyl, such as methyl, ethyl, propyl, isopropyl, etc).

In one embodiment, R₁ is selected from the group consisting of -NH₂, -NHC(=O)CH₃,

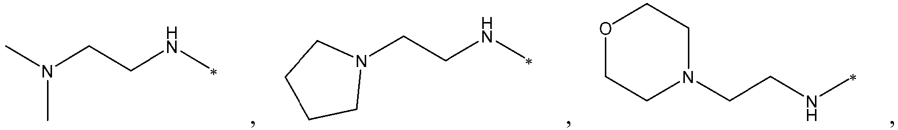

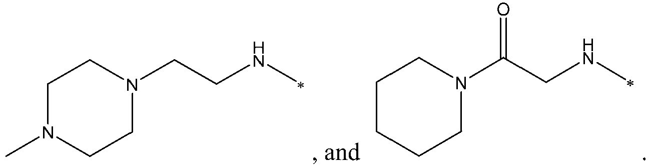

In one embodiment, R₁ is selected from the group consisting of OR₁₁, R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C_{5~20} heteroaryl, substituted or unsubstituted C_{1~6} alkylacyl, substituted or unsubstituted C_{1~6} alkylsulfonyl, substituted or unsubstituted C_{5~20} heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C₅₋₂₀ heteroaryl, cyano, -NR₃₁R₃₂, -C(=O)R₃₃, hydroxyl, and mercapto;
preferably, R₁₁ is selected from the group consisting of hydrogen and substituted or unsubstituted C_{1~6} alkyl, the substituent is selected from the group consisting of -NR₃₁R₃₂, - C(=O)R₃₃, and substituted or unsubstituted C_{3~8} heterocyclyl.

In one embodiment, R₁ is selected from the group consisting of -C(=O)NHR₁₁, wherein R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C_{5~20} heteroaryl, substituted or unsubstituted C_{1~6} alkylacyl, substituted or unsubstituted C_{1~6} alkylsulfonyl, substituted or unsubstituted C_{5~20} heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, - C(=O)R₃₃, hydroxyl, and mercapto;
preferably, R₁₁ is selected from the group consisting of hydrogen and substituted or unsubstituted C_{1~6} alkyl, the substituent is selected from the group consisting of -NR₃₁R₃₂, - C(=O)R₃₃, and substituted or unsubstituted C_{3~8} heterocyclyl.

In the above embodiments involving R₁, R₃₁ and R₃₂ are each independently selected from the group consisting of hydrogen, sulfonamido, substituted or unsubstituted C_{1~6} alkyl, substituted or unsubstituted C₂₋₆ alkenyl, substituted or unsubstituted C₂₋₆ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, -C(=O)OR₃₄, and mercapto;
R₃₃ is selected from the group consisting of hydrogen, substituted or unsubstituted C_{1~8} alkyl, substituted or unsubstituted C_{1~8} alkoxy, substituted or unsubstituted C_{3~8} cycloalkyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃₄ is selected from the group consisting of hydrogen, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto.

In the above embodiments involving R₁, R₃₁ and R₃₂ can be each independently hydrogen, substituted or unsubstituted C_{1~6} alkyl, and substituted or unsubstituted C_{3~8} heterocyclyl (the substituent can be C_{1~6} alkyl, such as methyl, ethyl, propyl, isopropyl, etc). R₃₃ can be each independently substituted or unsubstituted C_{1~6} alkyl, and substituted or unsubstituted C_{3~8} heterocyclyl (the substituent can be C_{1~6} alkyl, such as methyl, ethyl, propyl, isopropyl, etc).

In one embodiment, R₁ is -NH₂, and R₂ is hydrogen.

In one embodiment, R₄ and R₅ are each independently selected from the group consisting of hydrogen, halogen, -NR₃₁R₃₂, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto. In one embodiment, R₄ and R₅ are both hydrogen.

In one embodiment, R₆ and R₇ are hydrogen;
R₈ is selected from the group consisting of substituted or unsubstituted cyclopropyl, substituted or unsubstituted cyclobutyl, substituted or unsubstituted cyclohexyl, and substituted or unsubstituted bicyclic carbon ring; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, oxo, carboxyl, and mercapto.

In one embodiment, R₆ and R₇ are hydrogen; R₈ is selected from the group consisting of substituted or unsubstituted C₅-C₁₂ bicyclic carbon ring, the bicyclic carbon ring is selected from the group consisting of C₅-C₁₂ spirocyclic carbon ring, C₅-C₁₂ fused carbon ring, and C₅-C₁₂ bridged carbon ring; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, oxo, carboxyl, and mercapto.

In one embodiment, the bicyclic carbon ring is selected from the following structural formulas:

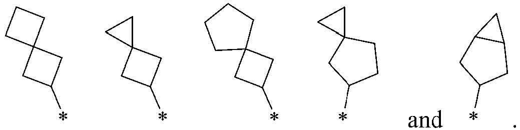

In one embodiment, R₆ and R₇ are hydrogen; R₈ is selected from the group consisting of substituted or unsubstituted cyclopropyl, substituted or unsubstituted cyclobutyl, substituted or unsubstituted cyclohexyl, and the substituent is selected from the group consisting of C_{1~6} alkyl (such as methyl, ethyl, propyl, isopropyl, or tert-butyl). Preferably, R₈ is cyclopropyl; or, R₈ is cyclohexyl; or, R₈ is selected from the group consisting of cyclobutyl, 3-methyl-cyclobutan-1-yl, and 3,3-dimethyl-cyclobutan-1-yl.

In one embodiment, R₆ is hydrogen, R₈ and the C atom to which R₈ is connected, together with R₇ and the C atom to which R₇ is connected, form a 5- to 8-membered carbon ring. In one embodiment, the 5- to 8-membered carbon ring is selected from the group consisting of cyclopentane ring, and cyclohexane ring.

In one embodiment, R₆ and R₈, together with the C atom to which R₆ and R₈ are connected, form a 4- to 8-membered monocyclic or polycyclic ring system; R₇ is hydrogen. In one embodiment, the R₆ and R₈, together with the C atom to which R₆ and R₈ are connected, form a 4- to 8-membered monocyclic ring system, for example C₄₋₈ cycloalkane ring, or C_{3~8} heterocyclic structure. In one embodiment, the 4- to 8-membered monocyclic or polycyclic ring system can be cyclobutane ring, cyclopentane ring, cyclohexane ring, azacyclobutane ring, azacyclopentane ring, azacyclohexane ring, oxetane ring, oxolane ring, oxepane ring, thietane ring, thiolane ring, thiane ring, etc. These 4- to 8-membered monocyclic or polycyclic ring system can be optionally substituted with one or more R₄₁, for example, hydrogen, halogen, oxo, cyano, hydroxyl, substituted or unsubstituted C_{1~8} alkyl (such as methyl, ethyl, propyl, isopropyl, tert-butyl, etc), substituted or unsubstituted C_{1~8} haloalkyl, substituted or unsubstituted C_{1~8} alkoxy, or substituted or unsubstituted C_{3~8} cycloalkyl.

Preferably, the 4- to 8-membered monocyclic or polycyclic ring system is selected from the following structural formulas: 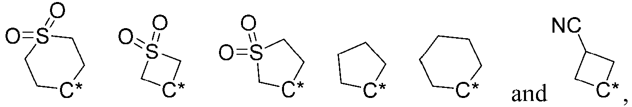 wherein C* represents the connection positions of the structural formulas, which is connected with two groups, one is the pyrazole ring structure and the other is the -CR₃R₇ structure.

In one embodiment, R₃ is cyano.

In one embodiment, the compound is selected from the following compounds:

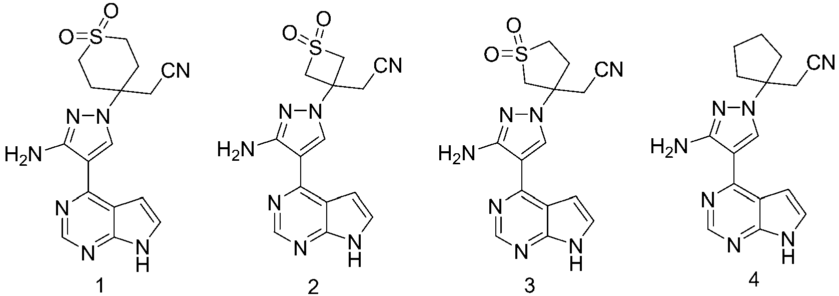

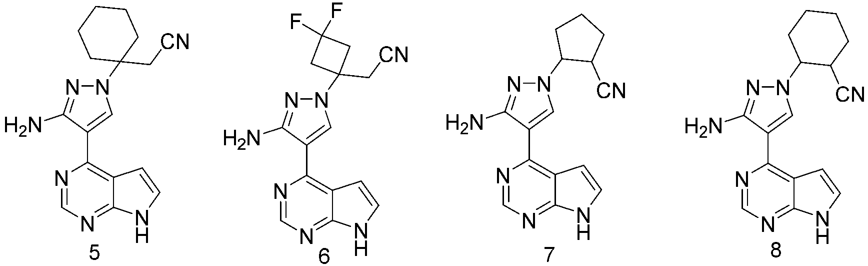

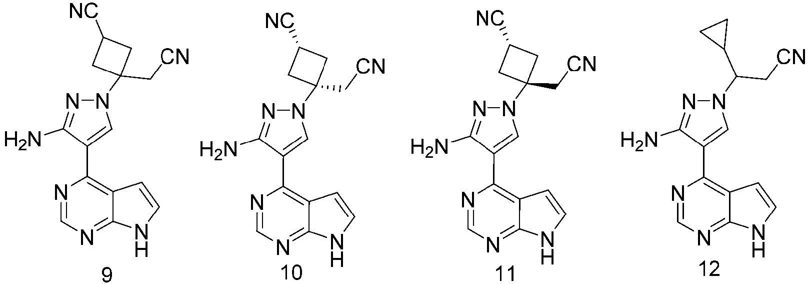

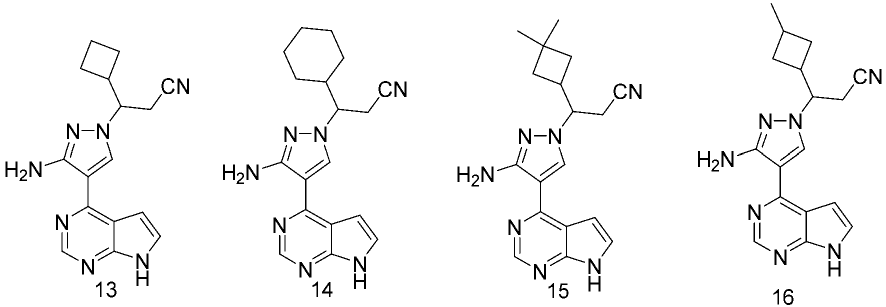

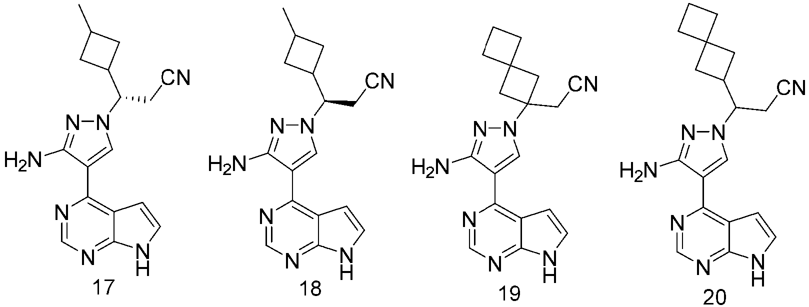

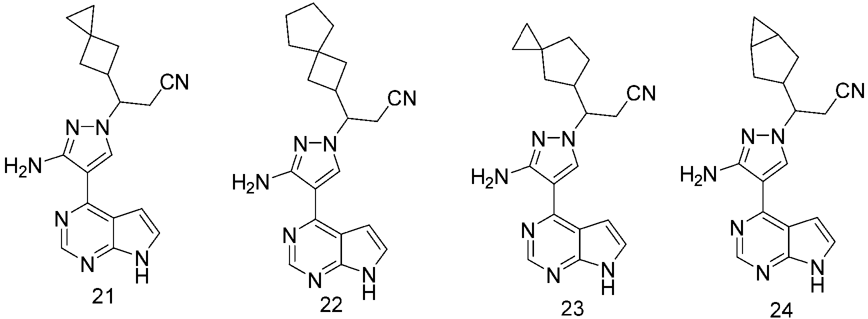

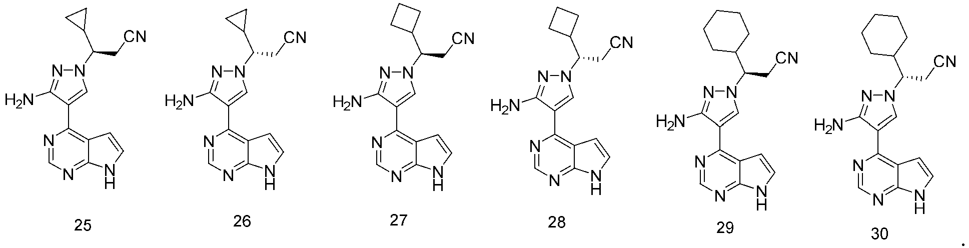

The compounds described in the present disclosure can be prepared by the following methods. The following methods and examples are to illustrate these methods. These procedures and examples should not be construed as limiting the present disclosure in any way. The compounds described herein can also be synthesized using standard synthesis techniques known to those skilled in the art, or methods known in the art and methods described herein can be used in combination.

The chemical reactions in the embodiments of the present disclosure are completed in a suitable solvent, and the solvent must be suitable for the chemical changes of the present disclosure and the reagents and materials required. In order to obtain the compounds of the present disclosure, it is sometimes necessary for those skilled in the art to modify or select the synthesis steps or reaction schemes based on the existing embodiments.

An important consideration in the planning of any synthetic route in the art is to select an appropriate protecting group for the reactive functional group (such as the amino group in the present disclosure). For trained practitioners, Greene and Wuts (Protective Groups In Organic Synthesis, Wiley and Sons, 1991) is the authority in this regard. All references cited in the present disclosure are incorporated into the present disclosure in their entirety.

The reactions described herein can be monitored according to any suitable method known in the art. For example, product formation can be monitored by a broad spectrum method such as nuclear magnetic resonance spectroscopy (such as ¹H or ¹³C), infrared spectroscopy, spectrophotometry (such as UV-visible light), mass spectrometry, etc., or chromatography such as high performance liquid chromatography (HPLC) or thin layer chromatography.

The compound of formula I of the present disclosure can be prepared by those skilled in the field of organic synthesis using standard methods in the art through the following procedures:

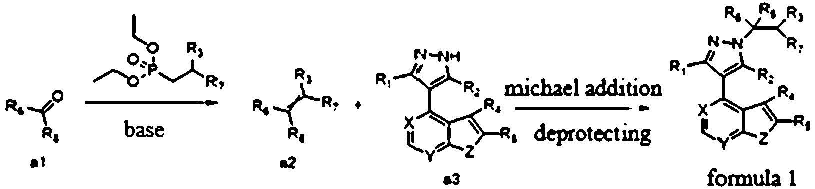

Aldehyde or ketone compound 1 undergoes Witting reaction with phosphate substituted by R₃ and R₇ to generate compound 2. Compound 2 undergoes Michael addition reaction with compound 3, and the protective group is removed to obtain compound I of the present invention. Partial chiral compounds are further separated by a chiral column (Chiral column: CHIRAL ART Amylose-C, NEO 3>25 cm, mobile phase A: CO₂, mobile phase B: IPA, flow rate 90 mL/min, gradient 50% B).

### Pharmaceutical composition and use thereof

The present invention also provides a pharmaceutical composition, which comprises the compound or pharmaceutically acceptable salts, solvates, active metabolites, polymorphs, isotopic markers, isomers or prodrugs thereof in any one of the above embodiments, and a pharmaceutically acceptable carrier.

The pharmaceutical composition comprises, but not limited to, oral, parenteral, topical and rectal dosage forms, etc. For example, the pharmaceutical composition can be in the form of a tablet, capsule, pill, powder, sustained release preparation, solution or suspension for oral administration; a sterile solution, suspension or emulsion for parenteral injection; an ointment, a cream, or a gel, etc. for external use; eye drops for external use; inhalants for external use; or a suppository for rectal administration.

The pharmaceutical composition can also comprise other active ingredients or pharmaceuticals, in combination with the compound or pharmaceutically acceptable salts, solvates, active metabolites, polymorphs, isotopic markers, isomers or prodrugs thereof.

The present invention also provides use of the above mentioned compound or pharmaceutically acceptable salts, solvates, active metabolites, polymorphs, isotopic markers, isomers or prodrugs thereof, as well as the above mentioned pharmaceutical composition in manufacture of pharmaceuticals for treating Rho kinase mediated diseases, wherein the Rho kinases can comprise types such as ROCK1 and ROCK2.

The present invention also relates to method for treating Rho kinase mediated diseases, which comprises administering a therapeutically effective amount of the above mentioned azacycloalkane compound or pharmaceutically acceptable salts, solvates, active metabolites, polymorphs, isotopic markers, isomers or prodrugs thereof, or the above mentioned pharmaceutical composition to a patient in need of administration, wherein the Rho kinases can comprise types such as ROCK1 and ROCK2.

In one embodiment, the Rho kinase mediated diseases are asthma, cancer, glaucoma, insulin resistance, kidney failure, neuronal degeneration, or osteoporosis.

The heterocyclic compound provided in the present disclosure have significant Rho-associated kinase inhibitory activity, the enzymatic activity and cell activity thereof are better than those of existing ROCK inhibitors such as Ripasudil, Netarsudil, and Belumosudil, and thus have great potential for application.

In order to clarify the purpose, technical solution, and advantages of the present disclosure, the technical solution of exemplary embodiments of the present disclosure will be further described below.

### Example 1: 2-(4-(3-amino-4-(7H-pyrrolo[2,3-d]pyrimidin-4-yl)-1H-pyrazol-1-yl)-1,1-dioxidotetrahydro-2H-thiopyran-4-yl)acetonitrile

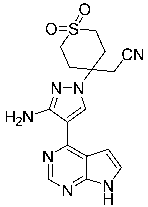

### Synthesis route:

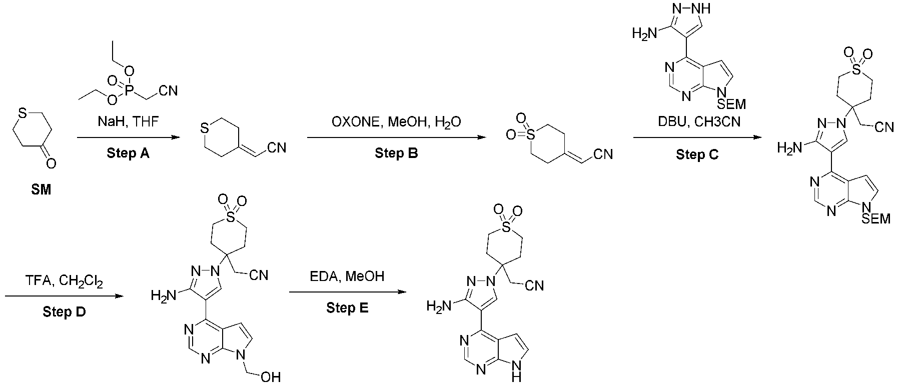

### Step A: 2-(tetrahydro-4H-thiopyran-4-ylidene)acetonitrile

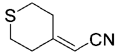

Sodium hydride (826 mg, 34.4 mmol, 2.0 eq) was added to a solution of diethyl (cyanomethyl)phosphonate (3.04 g, 17.2 mmol, 1.0 eq) in tetrahydrofuran (50 mL) at 0 °C. The reaction solution reacted at 0 °C for 0.5 h. Tetrahydro-4H-thiopyran-4-one (2 g, 17.2 mmol, 1.0eq) was added to the reaction solution. The temperature was raised to 25 °C and the reaction was continued for 2 h. After the reaction had been completed, the reaction solution was poured into the ammonium chloride aqueous solution (200 mL) and extraction was carried out with ethyl acetate (100 mL * 3). The organic phases were combined, dried over anhydrous sodium sulfate, and filtered. The filtrate was concentrated under reduced pressure. The residue was purified by column chromatography (petroleum ether: ethyl acetate=9:1) to obtain a colorless liquid product (2 g, yield=83%).

LC-MS(ESI), m/z: [M+H]⁺ =140.2.

### Step B: 2-(1,1-dioxidodihydro-4H-thiopyran-4-ylidene)acetonitrile

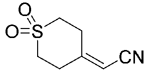

Potassium peroxymonosulfonate (4.98 g, 14.38 mmol, 2.0 eq) was added to the solution of 2-(tetrahydro-4H-thiopyran-4-ylidene)acetonitrile (1 g, 7.19 mmol, 1.0 eq) in methanol (20 mL) and water (10 mL) at room temperature. The reaction solution reacted at 25 °C for 7 h. After the reaction had been completed, the reaction solution was diluted with water (100 mL), and extraction was carried out with ethyl acetate (50 mL * 3). The organic phases were collected, dried over anhydrous sodium sulfate, and filtered. The filtrate was concentrated under reduced pressure to obtain a white solid product (900 mg, yield=73%).

LC-MS (ESI), m/z: [M+H]⁺ =172.1.

### Step C: 2-(4-(3-amino-4-(7-((2-(trimethylsilyl)ethoxy)methyl)-7H-pyrrolo[2,3-d]pyrimidin-4-yl)-1H-pyrazol-1-yl)-1,1-dioxotetrahydro-2H-thiopyran-4-yl acetonitrile

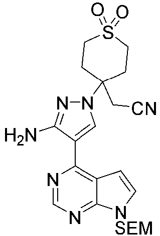

At romm temperature, a solution of 2-(1,1-dioxidodihydro-4H-thiopyran-4-ylidene)acetonitrile (342 mg, 2 mmol, 2.0eq), 4-(7-(2-trimethylsilylethoxymethyl)-7H-pyrrolopyrrolidin-4-yl pyrazole (330 mg, 1 mmol, 1.0eq), and 1,8-diazabicyclo[5.4.0]undec-7-ene (456 mg, 3 mmol, 1.5eq) in acetonitrile (20 mL) reacted at 25 °C for 16 h. After the reaction had been completed, the reaction solution was concentrated under reduced pressure. The residue was purified by column chromatography (petroleum ether: ethyl acetate=1:3) to obtain a light yellow solid product (360 mg, yield=72%).

LC-MS (ESI), m/z: [M+H]⁺ =502.2.

### Step D: 2-(4-(3-amino-4-(7-(methylol)-7H-pyrrolo[2,3-d]pyrimidin-4-yl)-1H-pyrazol-1-yl)-1,1- dioxidotetrahydro-2H-thiopyran-4-yl)acetonitrile

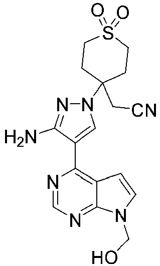

At room temperature, a solution of 2-(4-(3-amino-4-(7-((2-(trimethylsilyl)ethoxy)methyl)-7H-pyrrolo[2,3-d]pyrimidin-4-yl)-1H-pyrazol-1-yl)-1,1-dioxotetrahydro-2H-thiopyran-4-ylacetonitrile (300 mg, 0.6mmol, 1.0eq) in trifluoroacetic acid (3 mL) and dichloromethane (9 mL) was stirred at 25 °C for 2h. After the reaction had been completed, the crude product was obtained by vacuum concentration, which was directly used for the next step.

LC-MS (ESI), m/z: [M+H]⁺ = 402.1.

### Step E: 2-(4-(3-amino-4-(7H-pyrrolo[2,3-d]pyrimidin-4-yl)-1H-pyrazol-1-yl)-1,1-dioxidotetrahydro-2H-thiopyran-4-yl)acetonitrile

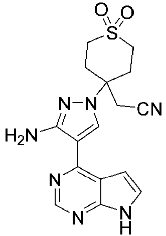

At room temperature, ethylenediamine (1 mL) was added to a solution of 2-(4-(3-amino-4-(7-(methylol)-7H-pyrrolo[2,3-d]pyrimidin-4-yl)-1H-pyrazol-1-yl)-1,1-dioxidotetrahydro-2H-thiopyran-4-yl)acetonitrile in methanol (10 mL). The reaction solution was stirred at 25 °C for 0.5 h. After the reaction had been completed, the reaction solution was concentrated under reduced pressure. The residue was purified by column chromatography (dichloromethane: methanol=10:1) to obtain a white solid product (97 mg, yield=36%).

LC-MS (ESI), m/z: [M+H]⁺ = 372.1.

¹H-NMR (400 MHz, DMSO-*d₆*) δ 12.07 (s, 1H), 8.67 (s, 1H), 8.56 (s, 1H), 7.55 (dd, J = 3.6, 2.4 Hz, 1H), 7.09 (dd, J = 3.6, 2.0 Hz, 1H), 6.36 (s, 2H), 3.31 - 3.21 (m, 4H), 3.13 - 3.02 (m, 4H), 2.48 - 2.42 (m, 2H).

The following examples were prepared according to the experimental route and method in Example 1:

| | |
|---|---|
| Example 2: 2-(3-(3-amino-4-(7H-pyrrolo[2,3-d]pyrimidin-4-yl)-1H-pyrazol-1-yl)-1,1-dioxothietan-3-yl)acetonitrile | |
| 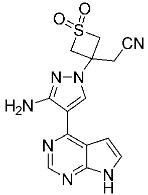 | LC-MS(ESI), m/z: [M+H]⁺ =344. |
| Example 3: 2-(3-(3-amino-4-(7H-pyrrolo[2,3-d]pyrimidin-4-yl)-1H-pyrazol-1-yl)-1,1-dioxotetrahydrothiophen-3-yl)acetonitrile | |
| 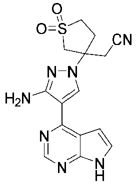 | LC-MS(ESI), m/z: [M+H]⁺ =358. |
| Example 4: 2-(1-(3-amino-4-(7H-pyrrolo[2,3-d]pyrimidin-4-yl)-1H-pyrazol-1-yl)cyclopentyl)acetonitrile | |
| 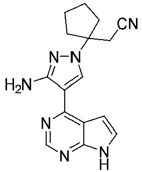 | LC-MS (ESI), m/z: [M+H]⁺ = 308.0. |
| | ¹H-NMR(400MHz, DMSO-*d*6) δ 12.14 (s, 1H), 8.67 (s, 1H), 8.41 (s, 1H), 7.59 - 7.53 (m, 1H), 7.05 (dd, J = 3.5, 1.7 Hz, 1H), 3.27 - 3.20 (m, 2H), 2.54 (dd, J = 12.4, 6.2 Hz, 2H), 1.96 (dt, J = 13.3, 6.6 Hz, 2H), 1.77 (d, J = 7.1 Hz, 2H), 1.69 (dd, J = 7.2, 4.1 Hz, 2H). |
| Example 5: 2-(1-(3-amino-4-(7H-pyrrolo[2,3-d]pyrimidin-4-yl)-1H-pyrazol-1-yl)cyclohexyl)acetonitrile | |
| 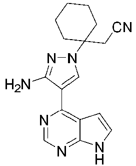 | LC-MS(ESI), m/z: [M+H]⁺ =322.0. |
| | ¹H-NMR (400 MHz, DMSO-d6) δ 12.03 (s, 1H), 8.64 (s, 1H), 8.37 (s, 1H), 7.52 (d, *J = 3.0* Hz, 1H), 6.98 (d, *J =* 3.4 Hz, 1H), 6.24 (s, 2H), 3.07 (s, 1H), 2.48-2.47 (m, 2H), 1.86-1.81 (m, 2H), 1.58-1.56 (m, 2H), 1.49-1.40 (m, 4H). |
| Example 6: 2-(1-(3-amino-4-(7H-pyrrolo[2,3-d]pyrimidin-4-yl)-1H-pyrazol-1-yl)-3,3-difluorocyclobutyl)acetonitrile | |
| 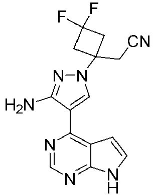 | LC-MS (ESI), m/z: [M+H]⁺ =330. |
| Example 7: 2-(3-amino-4-(7H-pyrrolo[2,3-d]pyrimidin-4-yl)-1H-pyrazol-1-yl)cyclopentane-1-nitrile | |
| 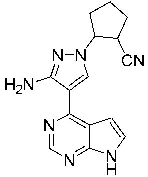 | LC-MS(ESI), m/z: [M+H]⁺ =294. |
| Example 8: 2-(3-amino-4-(7H-pyrrolo[2,3-d]pyrimidin-4-yl)-1H-pyrazol-1-yl)cyclohexane-1-nitrile | |
| 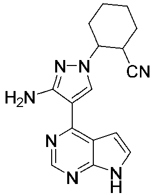 | LC-MS (ESI), m/z: [M+H]⁺ = 450.8. |
| | ¹H-NMR(400MHz, CD₃OD) δ 8.72 (s, 1H), 8.13 (s, 1H), 7.60 (d, J = 3.6 Hz, 1H), 7.07 (d, J = 3.7 Hz, 1H), 4.50 (td, J = 11.2, 3.9 Hz, 1H), 3.29 (s, 1H), 2.34-2.31 (m, 1H), 2.08 - 2.02 (m, 1H), 1.97 - 1.79 (m, 4H), 1.64 - 1.55 (m, 1H), 1.44-1.39 (m, 1H). |
| Example 9: 3-(3-amino-4-(7H-pyrrolo[2,3-d]pyrimidin-4-yl)-1H-pyrazol-1-yl)-3-(cyanomethyl)cyclobutane-1-carbonitrile | |
| 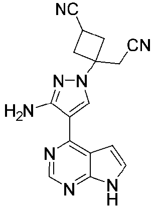 | LC-MS (ESI), m/z: [M+H]⁺ = 319.0. |
| Example 10: (1r,3r)-3-(3-amino-4-(7H-pyrrolo[2,3-d]pyrimidin-4-yl)-1H-pyrazol-1-yl)-3-(cyanomethyl)cyclobutane-1-carbonitrile | |
| 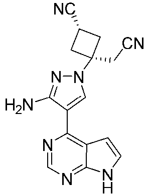 | LC-MS(ESI), m/z: [M+H]⁺ =319.0. |
| | ¹H-NMR(400MHz, DMSO-*d*6) δ 8.65 (s, 1H), 8.48 (s, 1H), 7.55 (m, 1H), 7.06 (d, J = 3.6 Hz, 1H), 3.42 (s, 2H), 3.08 (m, 2H), 2.71 (m, 2H). |
| Example 11: (1s,3s)-3-(3-amino-4-(7H-pyrrolo[2,3-d]pyrimidin-4-yl)-1H-pyrazol-1-yl)-3-(cyanomethyl)cyclobutane -1-carbonitrile | |
| 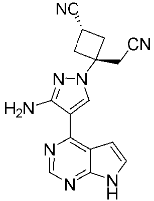 | LC-MS(ESI), m/z: [M+H]⁺ =319.0. |
| | ¹H-NMR(400MHz, DMSO-*d*6) δ 8.65 (s, 1H), 8.53 (s, 1H), 7.55 (m, 1H), 7.06 (d, J = 3.4 Hz, 1H), 3.35 (s, 2H), 3.17 (m, 2H), 2.76 (m, 2H). |
| Example 12: 3-(3-amino-4-(7H-pyrrolo[2,3-d]pyrimidin-4-yl)-1H-pyrazol-1-yl)-3-cyclopropylpropanitrile | |
| 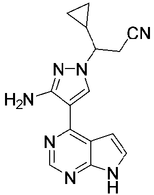 | LC-MS (ESI), m/z: [M+H]⁺ =294.0. |
| | ¹H-NMR (400 MHz, DMSO-*d*6) δ 12.28 (s, 1H), 8.70 (s, 1H), 8.62 (s, 1H), 7.61 (dd, J = 3.5, 2.4 Hz, 1H), 6.94 (dd, J = 3.6, 1.7 Hz, 1H), 6.55 - 6.15 (m, 2H), 3.86 - 3.75 (m, 1H), 3.24 (dd, J = 10.8, 6.9 Hz, 2H), 1.39-1.33 (m, 1H), 0.69-0.66 (m, 1H), 0.50-0.48 (m, 3H). |
| Example 13: 3-(3-amino-4-(7H-pyrrolo[2,3-d]pyrimidin-4-yl)-1H-pyrazol-1-yl)-3-cyclobutylpropionitrile | |
| 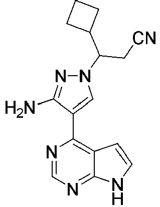 | LC-MS (ESI), m/z: [M+H]⁺ =308.15. |
| | ¹H-NMR (400 MHz, DMSO-d6) δ 12.02 (s, 1H), 8.63 (s, 1H), 8.60 (s, 1H), 7.53 (d, J = 4.0 Hz, 1H), 6.98 (d, J = 4.0 Hz, 1H), 6.23 (s, 2H), 4.49-4.43 (m, 1H), 3.01-3.00 (m, 2H), 2.80-2.76 (m, 1H), 2.09-2.05 (m, 1H), 1.86-1.79 (m, 5H). |
| Example 14: 3-(3-amino-4-(7H-pyrrolo[2,3-d]pyrimidin-4-yl)-1H-pyrazol-1-yl)-3-cyclohexylpropionitrile | |
| 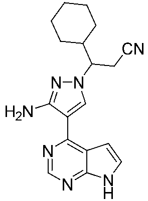 | LC-MS (ESI), m/z: [M+H]⁺ =336.10_{∘} |
| | ¹H-NMR (400 MHz, DMSO-*d*6) δ 12.02 (s, 1H), 8.63 (s, 1H), 8.59 (s, 1H), 7.52 (d, J = 4.0 Hz, 1H), 6.90 (d, J = 4.0 Hz, 1H), 6.25 (s, 2H), 4.25-4.20 (m, 1H), 3.21-3.08 (m, 2H), 1.83-1.08 (m, 11H)_{∘} |
| Example 15: 3-(3-amino-4-(7H-pyrrolo[2,3-d]pyrimidin-4-yl)-1H-pyrazol-1-yl)-3-(3,3-dimethylcyclobutyl)propionitrile | |
| 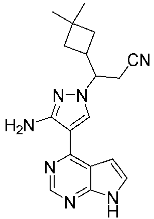 | LC-MS (ESI), m/z: [M+H]⁺ =336.10. |
| | ¹H-NMR (400 MHz, DMSO-*d*6) δ 12.02 (s, 1H), 8.62 (s, 1H), 8.59 (s, 1H), 7.55 - 7.50 (m, 1H), 6.92 (dd, *J* = 3.4, 1.2 Hz, 1H), 6.23 (s, 2H), 4.48 - 4.34 (m, 1H), 3.04 - 2.93 (m, 2H), 2.78 - 2.64 (m, 1H), 1.92 - 1.79 (m, 1H), 1.64 (dd, *J* = 19.9, 9.4 Hz, 2H), 1.58 - 1.49 (m, 1H), 1.10 (s, 3H), 1.04 (s, 3H). |
| Example 16: 3-(3-amino-4-(7H-pyrrolo[2,3-d]pyrimidin-4-yl)-1H-pyrazol-1-yl)-3-(3-methylcyclobutyl)propanitrile | |
| 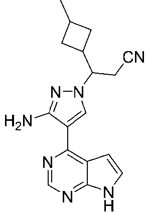 | LC-MS (ESI), m/z: [M+H]⁺ =322.10. |
| | ¹H-NMR (400 MHz, DMSO-*d*6) δ 12.02 (s, 1H), 8.73 - 8.46 (m, 2H), 7.55-7.49 (m, 1H), 6.94-6.89 (m, 1H), 6.23 (s, 2H), 4.56 - 4.31 (m, 1H), 3.04 - 2.97 (m, 1H), 2.84 - 2.56 (m, 1H), 2.39 - 1.83 (m, 3H), 1.78 - 1.33 (m, 2H), 1.15 - 0.91 (m, 3H). |
| Example 17: (3S)-3-(3-amino-4-(7H-pyrrolo[2,3-d]pyrimidin-4-yl)-1H-pyrazol-1-yl)-3-(-3-methylcyclobutyl)propanitrile | |
| 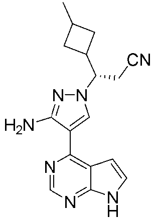 | LC-MS (ESI), m/z: [M+H]⁺ =322.10. |
| | ¹H-NMR (400 MHz, DMSO-*d*6) δ 12.02 (s, 1H), 8.66 - 8.53 (m, 2H), 7.57 - 7.49 (m, 1H), 6.92 (dd, *J =* 3.4, 1.7 Hz, 1H), 6.23 (s, 2H), 4.59 - 4.24 (m, 1H), 3.12 - 2.92 (m, 2H), 2.85 - 2.51 (m, 1H), 2.39 - 1.84 (m, 3H), 1.78 - 1.32 (m, 2H), 1.11 - 0.95 (m, 3H). |
| Example 18: (3R)-3-(3-amino-4-(7H-pyrrolo[2,3-d]pyrimidin-4-yl)-1H-pyrazol-1-yl)-3-(-3-methylcyclobutyl)propanitrile | |
| 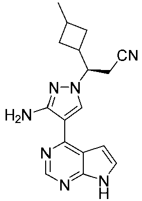 | LC-MS (ESI), m/z: [M+H]⁺ =322.10. |
| | ¹H-NMR (400 MHz, DMSO-*d*6) δ 12.02 (s, 1H), 8.67 - 8.53 (m, 2H), 7.56 - 7.50 (m, 1H), 6.92 (dd, *J* = 3.4, 1.7 Hz, 1H), 6.23 (s, 2H), 4.55 - 4.31 (m, 1H), 3.06 - 2.92 (m, 2H), 2.84 - 2.54 (m, 1H), 2.37 - 1.83 (m, 3H), 1.77 - 1.32 (m, 2H), 1.11 - 0.94 (m, 3H). |
| Example 19: 2-(2-(3-amino-4-(7H-pyrrolo[2,3-d]pyrimidin-4-yl)-1H-pyrazol-1-yl)spiro[3.3]heptan-2-yl)acetonitrile | |
| 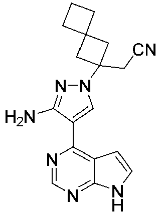 | LC-MS (ESI), m/z: [M+H]⁺ =334.15. |
| Example 20: 3-(3-amino-4-(7H-pyrrolo[2,3-d]pyrimidin-4-yl)-1H-pyrazol-1-yl)-3-(spiro[3.3]heptan-2-yl)propionitrile | |
| 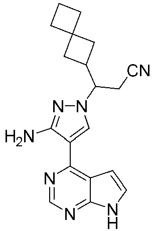 | LC-MS (ESI), m/z: [M+H]⁺ =348.2. |
| | ¹H-NMR (400 MHz, DMSO-*d*6) δ 12.02 (s, 1H), 8.63 (s, 1H), 8.58 (s, 1H), 7.53 (dd, *J =* 3.4, 2.5 Hz, 1H), 6.92 (dd*, J =* 3.5, 1.7 Hz, 1H), 6.23 (s, 2H), 4.40 - 4.30 (m, 1H), 3.06 - 2.94 (m, 2H), 2.69 - 2.52 (m, 1H), 2.16 - 2.07 (m, 1H), 2.03 - 1.90 (m, 2H), 1.89 - 1.67 (m, 7H). |
| Example 21: 3-(3-amino-4-(7H-pyrrolo[2,3-d]pyrimidin-4-yl)-1H-pyrazol-1-yl)-3-(spiro[2.3]hexan-5-yl)propionitrile | |
| 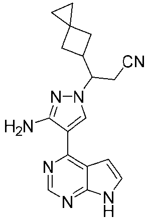 | LC-MS (ESI), m/z: [M+H]⁺ =334.15_{∘} |
| Example 22: 3-(3-amino-4-(7H-pyrrolo[2,3-d]pyrimidin-4-yl)-1H-pyrazol-1-yl)-3-(spiro[3.4]octan-2-yl)propionitrile | |
| 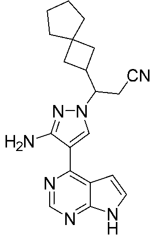 | LC-MS (ESI), m/z: [M+H]⁺ =362.20. |
| Example 23: 3-(3-amino-4-(7H-pyrrolo[2,3-d]pyrimidin-4-yl)-1H-pyrazol-1-yl)-3-(spiro[2.4]hept-5-yl)propionitrile | |
| 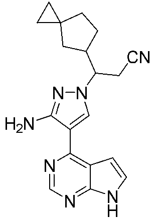 | LC-MS (ESI), m/z: [M+H]⁺ =348.20. |
| Example 24: 3-(3-amino-4-(7H-pyrrolo[2,3-d]pyrimidin-4-yl)-1H-pyrazol-1-yl)-3-(bicyclo[3.1.0]hexyl-3-yl)propionitrile | |
| 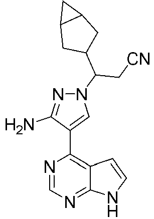 | LC-MS (ESI), m/z: [M+H]⁺ =334.20. |
| Example 25: (R)-3-(3-amino-4-(7H-pyrrolo[2,3-d]pyrimidin-4-yl)-1H-pyrazol-1-yl)-3-cyclopropylpropionitrile | |
| 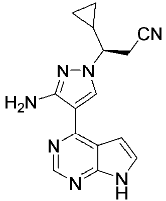 | LC-MS (ESI), m/z: [M+H]⁺ =294.0. |
| | ¹H-NMR (400 MHz, DMSO-*d*6) δ 12.28 (s, 1H), 8.70 (s, 1H), 8.62 (s, 1H), 7.61 (dd, J = 3.5, 2.4 Hz, 1H), 6.94 (dd, J = 3.6, 1.7 Hz, 1H), 6.55 - 6.15 (m, 2H), 3.86 - 3.75 (m, 1H), 3.24 (dd, J = 10.8, 6. Hz, 2H), 1.39-1.33 (m, 1H), 0.69-0.66 (m, 1H), 0.50-0.48 (m, 3H). |
| Example 26: (S)-3-(3-amino-4-(7H-pyrrolo[2,3-d]pyrimidin-4-yl)-1H-pyrazol-1-yl)-3-cyclopropylpropionitrile | |
| 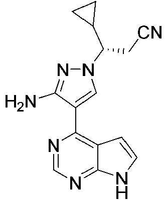 | LC-MS (ESI), m/z: [M+H]⁺ =294.0. |
| | ¹H-NMR (400 MHz, DMSO-*d*6) δ 12.28 (s, 1H), 8.70 (s, 1H), 8.62 (s, 1H), 7.61 (dd, J = 3.5, 2.4 Hz, 1H), 6.94 (dd, J = 3.6, 1.7 Hz, 1H), 6.55 - 6.15 (m, 2H), 3.86 - 3.75 (m, 1H), 3.24 (dd, J = 10.8, 6.9 Hz, 2H), 1.39-1.33 (m, 1H), 0.69-0.66 (m, 1H), 0.50-0.48 (m, 3H). |
| Example 27: (R)-3-(3-amino-4-(7H-pyrrolo[2,3-d]pyrimidin-4-yl)-1H-pyrazol-1-yl)-3-cyclobutylpropionitrile | |
| 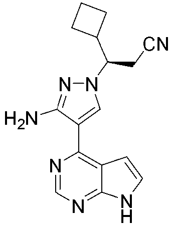 | LC-MS (ESI), m/z: [M+H]⁺ =308.15. |
| | ¹H-NMR (400 MHz, DMSO-d6) δ 12.02 (s, 1H), 8.63 (s, 1H), 8.60 (s, 1H), 7.53 (d, J = 4.0 Hz, 1H), 6.98 (d, J = 4.0 Hz, 1H), 6.23 (s, 2H), 4.49-4.43 (m, 1H), 3.01-3.00 (m, 2H), 2.80-2.76 (m, 1H), 2.09-2.05 (m, 1H), 1.86-1.79 (m, 5H). |
| Example 28: (S)-3-(3-amino-4-(7H-pyrrolo[2,3-d]pyrimidin-4-yl)-1H-pyrazol-1-yl)-3-cyclobutylpropionitrile | |
| 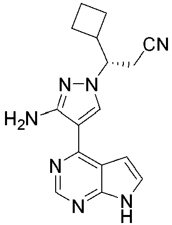 | LC-MS (ESI), m/z: [M+H]⁺ =308.15. |
| | ¹H-NMR (400 MHz, DMSO-d6) δ 12.02 (s, 1H), 8.63 (s, 1H), 8.60 (s, 1H), 7.53 (d, J = 4.0 Hz, 1H), 6.98 (d, J = 4.0 Hz, 1H), 6.23 (s, 2H), 4.49-4.43 (m, 1H), 3.01-3.00 (m, 2H), 2.80-2.76 (m, 1H), 2.09-2.05 (m, 1H), 1.86-1.79 (m, 5H). |
| Example 29: (R)-3-(3-amino-4-(7H-pyrrolo[2,3-d]pyrimidin-4-yl)-1H-pyrazol-1-yl)-3-cyclohexylpropionitrile | |
| 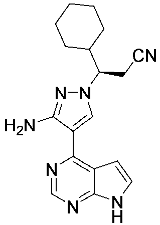 | LC-MS (ESI), m/z: [M+H]⁺ =336.10. |
| | ¹H-NMR (400 MHz, DMSO-*d*6) δ 12.02 (s, 1H), 8.63 (s, 1H), 8.59 (s, 1H), 7.52 (d, J = 4.0 Hz, 1H), 6.90 (d, J = 4.0 Hz, 1H), 6.25 (s, 2H), 4.25-4.20 (m, 1H), 3.21-3.08 (m, 2H), 1.83-1.08 (m, 11H). |
| Example 30: (S)-3-(3-amino-4-(7H-pyrrolo[2,3-d]pyrimidin-4-yl)-1H-pyrazol-1-yl)-3-cyclohexylpropionitrile | |
| 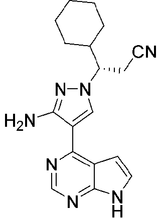 | LC-MS (ESI), m/z: [M+H]⁺ =336.10. |
| | ¹H-NMR (400 MHz, DMSO-*d*6) δ 12.02 (s, 1H), 8.63 (s, 1H), 8.59 (s, 1H), 7.52 (d, J = 4.0 Hz, 1H), 6.90 (d, J = 4.0 Hz, 1H), 6.25 (s, 2H), 4.25-4.20 (m, 1H), 3.21-3.08 (m, 2H), 1.83-1.08 (m, 11H). |

### Biological activity experiment

### 1. Detection of enzymatic activity (IC₅₀) of compounds

(1) ROCK1/2 detection experimental method
   (a) 50 nL of diluted compound working solution was transferred to each well of the reaction plate (784075, Greiner) using Echo 655.
   (b) the reaction plate was sealed with a sealing film and centrifugation was carried out at 1000g for 1 minute.
   (c) kinase solution was prepared.
   (d) 5 µL of kinase solution was added to each well of the reaction plate. The plate was sealed with a sealing film and centrifugation was carried out at 1000g for 30 seconds, then it was left at room temperature for 10 minutes.
   (e) a mixture of STK2 substrate biotin kinase and ATP was prepared.
   (f) 5 µL of the mixture of STK2 substrate biotin kinase and ATP was added to the reaction plate, centrifugation was carried out at 1000g for 30 seconds, and the reaction started.
   (g) ROCK1 kinase reacted at room temperature for 20 minutes, and ROCK2 kinase reacted at room temperature for 30 minutes.
   (h) a mixture of Sa-XL 665 (125 nM) and STK antibody Cryptate was prepared using HTRF detection buffer.
   (i) 10 µL of the mixture of Sa-XL 665 and STK antibody Cryptate was added to each well, centrifugation was carried out at 1000 g for 30 seconds, and the reaction was carried out at room temperature for 1 hour.

Envision 2104 was used to read 615 nm (Cryptate) and 665 nm (XL665) signals, and the signal intensity was used to characterize the activity level of the kinase.

The kinase activity data was expressed by comparing the kinase activity of the test compound and that of the blank group (containing only DMSO), and the IC₅₀ value was obtained by curve fitting using Prism software (GraphPad7.0).

The specific test results are shown in Table 1 below:

**Table 1: enzymatic activity of compounds**

| Example | IC₅₀ (nM) | |
|---|---|---|
| | ROCK1 | ROCK2 |
| 1 | <2 | <1 |
| 2 | <2 | <1 |
| 3 | <2 | <1 |
| 4 | <10 | <10 |
| 5 | <5 | <5 |
| 6 | <5 | <5 |
| 7 | <50 | <50 |
| 8 | <50 | <50 |
| 9 | <10 | <10 |
| 10 | <2 | <1 |
| 11 | <10 | <10 |
| 12 | <5 | <2 |
| 13 | <5 | <5 |
| 14 | <5 | <5 |
| 15 | <5 | <5 |
| 16 | <5 | <5 |
| 17 | <5 | <5 |
| 18 | <5 | <5 |
| 19 | <5 | <5 |
| 20 | <5 | <5 |
| 21 | <5 | <5 |
| 22 | <5 | <5 |
| 23 | <5 | <5 |
| 24 | <5 | <5 |
| 25 | <5 | <2 |
| 26 | <5 | <2 |
| 27 | <5 | <5 |
| 28 | <5 | <5 |
| 29 | <5 | <5 |
| 30 | <5 | <5 |
| Ripasudil | 22.02 | 29.62 |
| Netarsudil | 6.13 | 7.75 |
| Belumosudil | >1000 | 192 |

wherein Ripasudil has the following structural formula:

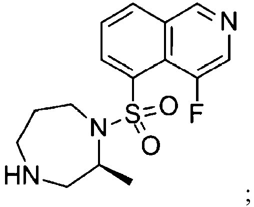

Netarsudil has the following structural formula:

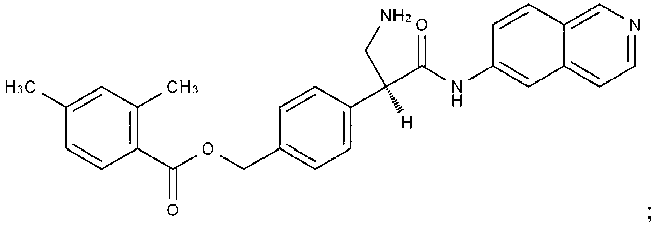

Belumosudil has the following structural formula:

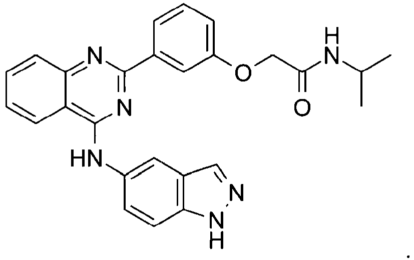

It can be seen from the data in the table that the enzymatic activity of the compounds in the embodiments of the present disclosure is better than that of the marketed ROCK inhibitors Ripasudil, Netarsudil, and Belumosudil. Some compounds exhibit very strong activity against the ROCK 1/2 kinase.

### 2. Cell Activity (IC₅₀) Detection of the compounds (In Cell Western for detecting phosphorylation of myosin light chain )

ROCK resulted in changes in the cytoskeleton by phosphorylating two amino acid sites of myosin light chain T18/S19. The rat smooth muscle cell line A7r5 was used, and the A7r5 cell culture conditions were DMEM containing 10% FBS. Phspho-MLC-T18/S19 specific antibody and a second detection antibody were used to detect the phosphorylation level of myosin light chain by In Cell Western. Cells treated with positive compounds were used as positive controls, while the group without compounds but with compound solvents was used as negative controls. DRAQ5 stained cell nuclei were used as internal references. GraphPad Prism 7.0 software was used, and fitting was carried out by using a non-linear regression curve with varying slopes to determine the absolute IC₅₀ value.

On the first day, A7r5 cells were resuspended in serum-free medium and seeded into 384 wells of PDL coated transparent black plates at a density of 5000 cells per well. Serum starvation culture was performed for 4 hours, and the cells were incubated with the compound in serum-free medium for 1 hour. 50 µL of 8% PFA (polyformaldehyde) was added to each well and fixed at room temperature for 1 hour. The liquid in the wells was discard and 90 µL of ice methanol was added to each well to permeabilize the cells at 4 °C for 1 hour. Then, PBST (0.1% Tween20-PBS) was added using an automatic separator to wash three times. After patting the plate for drying, 50 µL blocking solution was added to each well for sealing at room temperature for 1 hour. The phspho-MLC-T18/S19 specific antibody was diluted with the blocking solution at a ratio of 1:200. After adding 20 µL to each well, incubation was carried out overnight at 4 °C with a sealed film.

On the second day, the liquid in the wells was discarded and PBST was added using an automatic separator to wash the plate 5 times. After patting the plate for drying, the second detection antibody was diluted with blocking solution at a ratio of 1:800 and DRAQ5 at a ratio of 1:1000. After adding 20 µL to each well, incubation was carried out at room temperature for 1 hour. Then, the liquid in the wells was discarded and PBST was added using an automatic separator to wash the plate 3 times, followed by adding ddH2O to wash the plate 3 times. After patting the plate for drying, it was scaned using a LICOR Odyssey near-infrared imaging scanner.

The specific test results are shown in Table 2 below:

**Table 2: cellular activity of compounds**

| Example | IC₅₀(µM) | Example | IC₅₀(µM) | Example | IC₅₀(µM) |
|---|---|---|---|---|---|
| 1 | 0.092 | 11 | 0.466 | 21 | 0.185 |
| 2 | 0.085 | 12 | 0.085 | 22 | 0.264 |
| 3 | 0.078 | 13 | 0.122 | 23 | 0.195 |
| 4 | 0.160 | 14 | 0.175 | 24 | 0.214 |
| 5 | 0.111 | 15 | 0.215 | 25 | 0.075 |
| 6 | 0.102 | 16 | 0.284 | 26 | 0.088 |
| 7 | 1.860 | 17 | 0.225 | 27 | 0.095 |
| 8 | 1.943 | 18 | 0.296 | 28 | 0.120 |
| 9 | 0.068 | 19 | 0.167 | 29 | 0.106 |
| 10 | 0.048 | 20 | 0.218 | 30 | 0.182 |
| Ripasudil | 1.685 | Netarsudil | 0.288 | Belumosudil | 3.941 |

It can be seen from the data in the table that the compounds in the embodiments of the present disclosure have better cellular activity compared to the marketed ROCK inhibitors Ripasudil, Netarsudil, and Belumosudil. They have a stronger ability to induce changes in the cytoskeleton by phosphorylating two amino acid sites of myosin light chain T 18/S 19. Therefore, the application value of the compound of the present invention will be more extensive.

### 3. The role of compounds in a Norwegian rat glaucoma model induced by hypertonic saline solution

Experimental method: after animal anesthesia, the lateral canthus of the right eye was incised, and a polypropylene ring was placed at the equator of the eyeball, with the notch of the ring located in the upper quadrant of the eyeball, leaving only one scleral surface vein above. The bulbar conjunctiva was cut under a microscope, the scleral surface vein was expose, a microneedle was inserted into the vascular lumen towards the corneal edge and parallel to the vascular wall, and immediately, 50 µL of 1.85 M (10.8%) hypertonic saline (filtered through a filter with a pore size of 0.22 µm for backup use) was injected using a microsyringe at a rate of 2500 nL/sec. After injection, the polypropylene ring was removed and antibiotic eye ointment was applied to the operated eye. The control group of rats only underwent upper scleral vein puncture in the right eye and did not receive injection of hypertonic saline solution.

After modeling, a rebound tonometer was used continuously to measure the intraocular pressure of both eyes, once every 3 days, and the average of 6 measurements was took each time and recorded; After the intraocular pressure had been stabilized (about 20 days later), the subjects were randomly divided into three groups based on the most recent measurement of intraocular pressure and body weight: sham surgery group, model control group, and test drug group (0.02% Example 29) group, with 5 animals in each group. After grouping, eye drops were administered once a day, with one drop per time. During the administration period, dynamic intraocular pressure was measured once a week (detecting the intraocular pressure change curve from 0 to 24 hours after administration) until the end of the experiment.

Experimental results: the intraocular pressure change curve within 24 hours after administration shows that compared with the model control group, the intraocular pressure significantly decreased within 6 hours after administration, and gradually stabilized by 24 hours. The percentage decrease in intraocular pressure is shown in Figure 1.

The experimental results show that in the Norwegian rat glaucoma model induced by hypertonic saline, the compounds of the present invention have a strong effect on reducing animal intraocular pressure, and have the potential to treat ophthalmic diseases such as high intraocular pressure.

The above description combines preferred embodiments to explain the present application, but these embodiments are only exemplary and serve only illustrative purposes. On this basis, various substitutions and modifications can be made to the present application, all of which fall within the scope of protection of the present application.

## Claims

1. A heterocyclic compound shown in formula (I), or pharmaceutically acceptable salts, solvates, active metabolites, polymorphs, isotopic markers, isomers or prodrugs thereof: 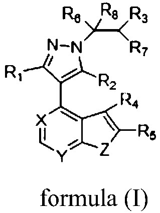
wherein X, Y and Z are each independently selected from the group consisting of C and N atoms, and X, Y and Z are not all C atoms at the same time;
R₁ is -NHR₁₁, -OR₁₁, -SR₁₁, or -C(=O)NHR₁₁; wherein R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C_{5~20} heteroaryl, substituted or unsubstituted C_{1~6} alkylacyl, substituted or unsubstituted C_{1~6} alkylsulfonyl, substituted or unsubstituted C₅₋₂₀ heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, -C(=O)R₃₃, hydroxyl, carboxyl, mercapto, substituted C_{3~8} cycloalkyl, substituted C_{3~8} heterocyclyl, substituted C_{6~20} aryl, and substituted C_{5~20} heteroaryl;
R₂ is selected from the group consisting of hydrogen, halogen, substituted or unsubstituted C_{1~6} alkyl, substituted or unsubstituted C₂₋₆ alkenyl, substituted or unsubstituted C₂₋₆ alkynyl, substituted or unsubstituted C_{1~6} haloalkyl, substituted or unsubstituted C_{1~6} alkoxy, cyano, amino, hydroxyl, carboxyl, and mercapto; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃ is selected from the group consisting of cyano, -CONH₂, and carboxyl;
R₄ and R₅ are each independently selected from the group consisting of hydrogen, halogen, -NR₃₁R₃₂, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
wherein (i) R₆ and R₈ are each independently selected from the group consisting of hydrogen, substituted or unsubstituted cyclopropyl, substituted or unsubstituted cyclobutyl, substituted or unsubstituted cyclohexyl, and substituted or unsubstituted bicyclic carbon ring; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, oxo, carboxyl, and mercapto; and R₆ and R₈ are not all hydrogen;
or, (ii) R₆ and R₈, together with the C atom to which R₆ and R₈ are connected, form a 4-to 8-membered monocyclic or polycyclic ring system; the 4- to 8-membered monocyclic or polycyclic ring system is optionally substituted with one or more R₄₁;
R₇ is selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{1~6} haloalkyl, substituted or unsubstituted C_{1~6} alkoxy, amino, hydroxyl, carboxyl, and mercapto;
or, R₆ is hydrogen, R₈ and the C atom to which R₈ is connected, together withR₇ and the C atom to which R₇ is connected, form a 5- to 8-membered carbon ring; the 5- to 8-membered carbon ring is optionally substituted with one or more R₄₁;
R₃₁ and R₃₂ are each independently selected from the group consisting of hydrogen, sulfonamido, substituted or unsubstituted C_{1~6} alkyl, substituted or unsubstituted C₂₋₆ alkenyl, substituted or unsubstituted C₂₋₆ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C₅₋₂₀ heteroaryl, cyano, hydroxyl, -C(=O)OR₃₄, and mercapto;
R₃₃ is selected from the group consisting of hydrogen, substituted or unsubstituted C_{1~8} alkyl, substituted or unsubstituted C_{1~8} alkoxy, substituted or unsubstituted C_{3~8} cycloalkyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃₄ is selected from the group consisting of hydrogen, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto;
R₄₁ is selected from the group consisting of hydrogen, halogen, oxo, cyano, hydroxyl, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto.

2. The heterocyclic compound or pharmaceutically acceptable salts, solvates, active metabolites, polymorphs, isotopic markers, isomers or prodrugs thereof according to claim 1, wherein X, Y and Z are all N atoms.

3. The heterocyclic compound or pharmaceutically acceptable salts, solvates, active metabolites, polymorphs, isotopic markers, isomers or prodrugs thereof according to claim 1, wherein R₁ is selected from the group consisting of -NHR₁₁, wherein R₁₁ is each independently selected from the group consisting of hydrogen, substituted or unsubstituted C_{1~6} alkyl, substituted or unsubstituted C₂₋₆ alkenyl, substituted or unsubstituted C₂₋₆ alkynyl, substituted or unsubstituted C_{3~6} cycloalkyl, substituted or unsubstituted C_{3~8} heterocyclyl, substituted or unsubstituted C_{6~20} aryl, substituted or unsubstituted C_{5~20} heteroaryl, substituted or unsubstituted C_{1~6} alkylacyl, substituted or unsubstituted C_{1~6} alkylsulfonyl, substituted or unsubstituted C_{5~20} heteroarylacyl, and substituted or unsubstituted C_{1~6} alkoxy; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, -C(=O)R₃₃, hydroxyl, mercapto, substituted C_{3~8} cycloalkyl, substituted C_{3~8} heterocyclyl, substituted C_{6~20} aryl, and substituted C_{5~20} heteroaryl;
preferably, R₁₁ is selected from the group consisting of hydrogen, substituted or unsubstituted C_{1~6} alkyl, and the substituent is selected from the group consisting of -NR₃₁R₃₂, - C(=O)R₃₃, and substituted or unsubstituted C_{3~8} heterocyclyl;
R₃₁ and R₃₂ are each independently selected from the group consisting of hydrogen, sulfonamido, substituted or unsubstituted C_{1~6} alkyl, substituted or unsubstituted C₂₋₆ alkenyl, substituted or unsubstituted C₂₋₆ alkynyl, substituted or unsubstituted C_{3~8} cycloalkyl, substituted or unsubstituted C_{3~8} heterocyclyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C₅₋₂₀ heteroaryl, cyano, hydroxyl, -C(=O)OR₃₄, and mercapto;
R₃₃ is selected from the group consisting of hydrogen, substituted or unsubstituted C_{1~8} alkyl, substituted or unsubstituted C_{1~8} alkoxy, substituted or unsubstituted C_{3~8} cycloalkyl, substituted or unsubstituted C_{6~20} aryl, and substituted or unsubstituted C_{5~20} heteroaryl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, carboxyl, and mercapto;
R₃₄ is selected from the group consisting of hydrogen, substituted or unsubstituted C_{1~8} alkyl, substituted or unsubstituted C_{1~8} haloalkyl, substituted or unsubstituted C_{1~8} alkoxy, and substituted or unsubstituted C_{3~8} cycloalkyl; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, hydroxyl, carboxyl, and mercapto.

4. The heterocyclic compound or pharmaceutically acceptable salts, solvates, active metabolites, polymorphs, isotopic markers, isomers or prodrugs thereof according to claim 3, wherein R₁₁ is selected from the group consisting of substituted or unsubstituted C_{1~6} alkyl, the substituent is selected from the group consisting of -NR₃₁R₃₂, wherein R₃₁ and R₃₂ are each independently hydrogen, substituted or unsubstituted C_{1~6} alkyl, or substituted or unsubstituted C_{3~8} heterocyclyl.

5. The heterocyclic compound or pharmaceutically acceptable salts, solvates, active metabolites, polymorphs, isotopic markers, isomers or prodrugs thereof according to claim 3, wherein R₁₁ is selected from the group consisting of substituted or unsubstituted C_{1~6} alkyl, the substituent is selected from the group consisting of -C(=O)R₃₃, wherein R₃₃ is independently substituted or unsubstituted C_{1~6} alkyl, or substituted or unsubstituted C_{3~8} heterocyclyl.

6. The heterocyclic compound or pharmaceutically acceptable salts, solvates, active metabolites, polymorphs, isotopic markers, isomers or prodrugs thereof according to claim 3, wherein
R₁ is selected from the group consisting of -NH₂, -NHC(=O)CH₃, 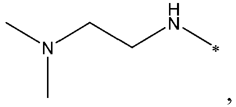 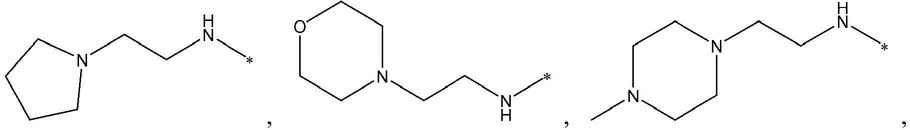 and

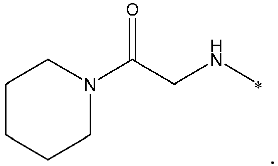

7. The heterocyclic compound or pharmaceutically acceptable salts, solvates, active metabolites, polymorphs, isotopic markers, isomers or prodrugs thereof according to claim 1, wherein R₁ is -NH₂, and R₂ is hydrogen.

8. The heterocyclic compound or pharmaceutically acceptable salts, solvates, active metabolites, polymorphs, isotopic markers, isomers or prodrugs thereof according to claim 1, wherein R₄ and R₅ are both hydrogen.

9. The heterocyclic compound or pharmaceutically acceptable salts, solvates, active metabolites, polymorphs, isotopic markers, isomers or prodrugs thereof according to claim 1, wherein R₆ and R₇ are hydrogen;
R₈ is selected from the group consisting of substituted or unsubstituted cyclopropyl, substituted or unsubstituted cyclobutyl, substituted or unsubstituted cyclohexyl, and substituted or unsubstituted bicyclic carbon ring; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, oxo, carboxyl, and mercapto.

10. The heterocyclic compound or pharmaceutically acceptable salts, solvates, active metabolites, polymorphs, isotopic markers, isomers or prodrugs thereof according to any one of claims 1 to 9, wherein R₈ is selected from the group consisting of substituted or unsubstituted C₅-C₁₂ bicyclic carbon ring, the bicyclic carbon ring is selected from the group consisting of C₅-C₁₂ spirocyclic carbon ring, C₅-C₁₂ fused carbon ring, and C₅-C₁₂ bridged carbon ring; the substituent is selected from the group consisting of halogen, C_{1~8} alkyl, C_{1~8} haloalkyl, C_{1~8} alkoxy, C_{3~8} cycloalkyl, C_{3~8} heterocyclyl, C_{6~20} aryl, C_{5~20} heteroaryl, cyano, -NR₃₁R₃₂, hydroxyl, oxo, carboxyl, and mercapto.

11. The heterocyclic compound or pharmaceutically acceptable salts, solvates, active metabolites, polymorphs, isotopic markers, isomers or prodrugs thereof according to claim 10, wherein the bicyclic carbon ring is selected from the following structural formulas:

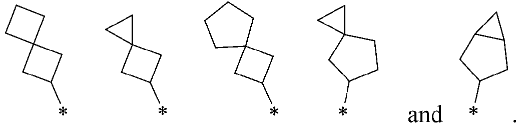

12. The heterocyclic compound or pharmaceutically acceptable salts, solvates, active metabolites, polymorphs, isotopic markers, isomers or prodrugs thereof according to any one of claims 1 to 10, wherein R₈ is selected from the group consisting of substituted or unsubstituted cyclopropyl, substituted or unsubstituted cyclobutyl, substituted or unsubstituted cyclohexyl, and the substituent is selected from the group consisting of C_{1~6} alkyl.

13. The heterocyclic compound or pharmaceutically acceptable salts, solvates, active metabolites, polymorphs, isotopic markers, isomers or prodrugs thereof according to claim 12, wherein R₈ is cyclopropyl;
or, R₈ is cyclohexyl;
or, R₈ is selected from the group consisting of cyclobutyl, 3-methyl-cyclobutan-1-yl, and 3,3-dimethyl-cyclobutan-1-yl.

14. The heterocyclic compound or pharmaceutically acceptable salts, solvates, active metabolites, polymorphs, isotopic markers, isomers or prodrugs thereof according to claim 1, wherein R₆ is hydrogen, R₈ and the C atom to which R₈ is connected, together with R₇ and the C atom to which R₇ is connected, form a 5- to 8-membered carbon ring.

15. The heterocyclic compound or pharmaceutically acceptable salts, solvates, active metabolites, polymorphs, isotopic markers, isomers or prodrugs thereof according to claim 14, wherein the 5- to 8-membered carbon ring is selected from the group consisting of cyclopentane ring, and cyclohexane ring,

16. The heterocyclic compound or pharmaceutically acceptable salts, solvates, active metabolites, polymorphs, isotopic markers, isomers or prodrugs thereof according to claim 1, wherein R₆ and R₈, together with the C atom to which R₆ and R₈ are connected, form a 4- to 8-membered monocyclic or polycyclic ring system;
R₇ is hydrogen.

17. The heterocyclic compound or pharmaceutically acceptable salts, solvates, active metabolites, polymorphs, isotopic markers, isomers or prodrugs thereof according to claim 16, wherein the 4- to 8-membered monocyclic or polycyclic ring system is selected from the following structural formulas: 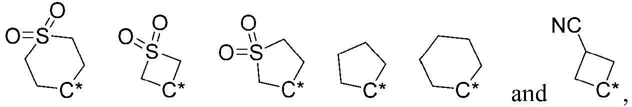 wherein C* represents the connection positions of the structural formulas.

18. The heterocyclic compound or pharmaceutically acceptable salts, solvates, active metabolites, polymorphs, isotopic markers, isomers or prodrugs thereof according to claim 1, wherein R₃ is cyano.

19. The heterocyclic compound or pharmaceutically acceptable salts, solvates, active metabolites, polymorphs, isotopic markers, isomers or prodrugs thereof according to claim 1, wherein the compound is selected from the following compounds:

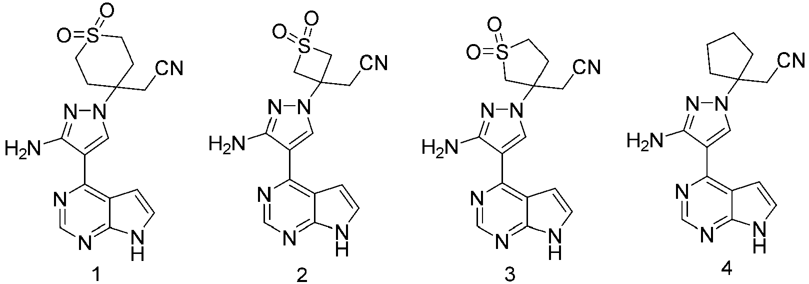

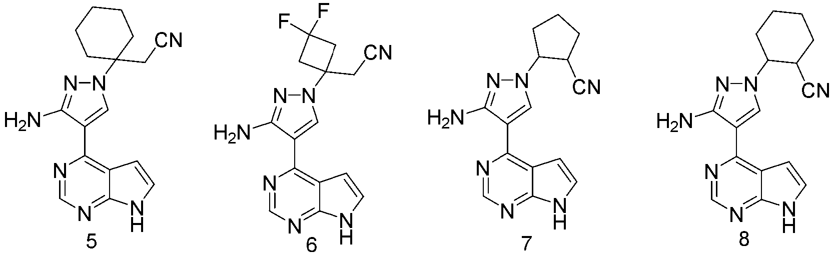

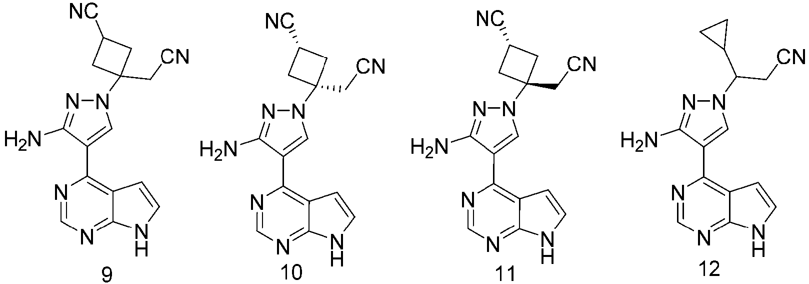

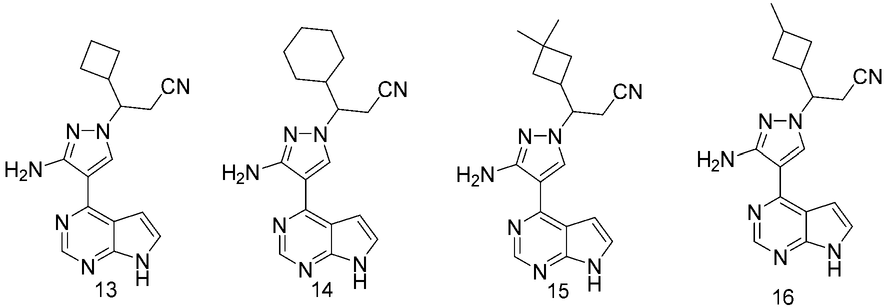

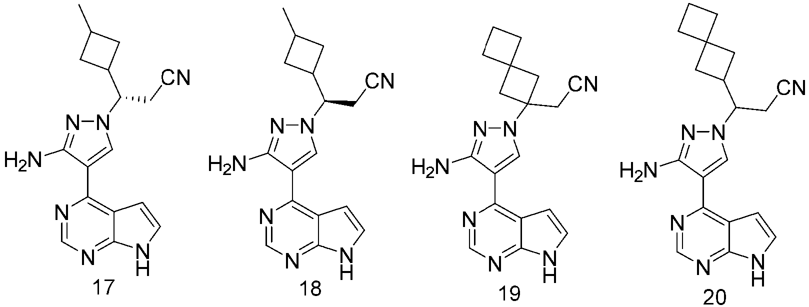

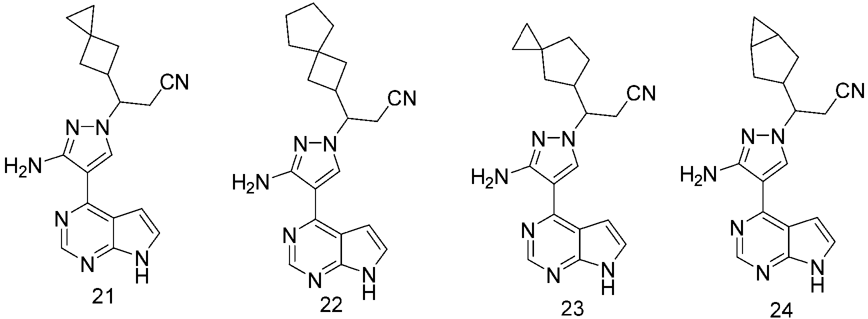

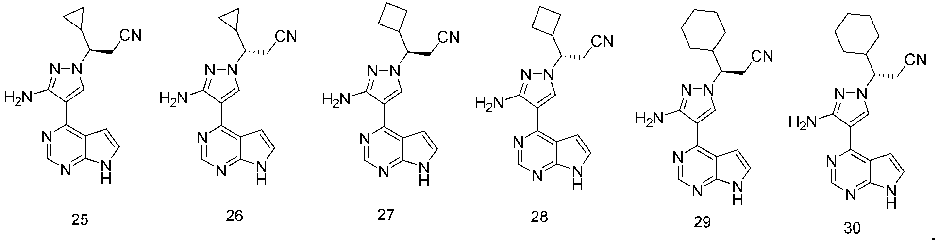

20. A pharmaceutical composition, comprising the heterocyclic compound or pharmaceutically acceptable salts, solvates, active metabolites, polymorphs, isotopic markers, isomers or prodrugs thereof according to any one of claims 1 to 19, and a pharmaceutically acceptable carrier.

21. Use of the heterocyclic compound or pharmaceutically acceptable salts, solvates, active metabolites, polymorphs, isotopic markers, isomers or prodrugs thereof according to any one of claims 1 to 19, and the pharmaceutical composition according to claim 20 in manufacture of pharmaceuticals for treating Rho kinase mediated diseases.

22. Use according to claim 21, wherein the Rho kinase mediated diseases are asthma, cancer, glaucoma, insulin resistance, kidney failure, neuronal degeneration, or osteoporosis.

23. A method for treating Rho kinase mediated diseases, comprising administering a therapeutically effective amount of the heterocyclic compound or pharmaceutically acceptable salts, solvates, active metabolites, polymorphs, isotopic markers, isomers or prodrugs thereof according to any one of claims 1 to 19, or the pharmaceutical composition according to claim 20 to a patient in need of administration.

24. The method according to claim 23, wherein the Rho kinase mediated diseases are asthma, cancer, glaucoma, insulin resistance, kidney failure, neuronal degeneration, or osteoporosis.
